# FASCICULE DE BREVET EUROPEEN

(11) **EP 0 893 985 B1**
(45) Date de publication et mention de la délivrance du brevet: **12.04.2000**
(21) Numéro de dépôt: 97915540.5
(22) Date de dépôt: 21.03.1997
(51) Int. Cl.: A61K 7/13

(54) **COMPOSITIONS DE TEINTURE DES FIBRES KERATINIQUES CONTENANT DES IMIDAZOLO-AZOLES; LEUR UTILISATION EN TEINTURE COMME COUPLEURS; PROCEDE DE TEINTURE**
ZUSAMMENSETZUNG ZUR FÄRBUNG VON KERATINISCHEN FASERN ENTHALTEND IMIDAZOLOAZOLE ALS KUPPLER, UND FÄRBEVERFAHREN
KERATIN FIBRE DYE COMPOSITIONS CONTAINING IMIDAZOLO-AZOLE COMPOUNDS, USE THEREOF AS DYE COUPLERS, AND DYEING METHOD

(30) Priorité: 22.03.1996 FR 9603628
(43) Date de publication de la demande: 03.02.1999
(73) Titulaire: L'OREAL, 75008 Paris (FR)
(72) Inventeur: VIDAL, Laurent, F-75013 Paris (FR); MALLE, Gérard, F-77100 Meaux (FR)
(74) Mandataire: Goulard, Sophie
(86) Numéro de dépôt international: FR9700508
(87) Numéro de publication internationale: WO9735552

(56) Documents cités:
- EP-A- 0 030 680
- WO-A-92/04883
- DE-A- 4 009 097
- DE-A- 4 133 957

## Description

L'invention a pour objet une composition pour la teinture par oxydation des fibres kératiniques en particulier des cheveux humains contenant au moins un composé imidazoloazole comme coupleur et au moins une base d'oxydation.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les méta-aminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation, permanente, transpiration, frottements).

Les colorants doivent également permettre de couvrir les cheveux blancs, et être enfin les moins sélectifs possible, c'est-à-dire permettre d'obtenir des écarts de coloration les plus faibles possible tout au long d'une même fibre kératinique, qui peut être en effet, différemment sensibilisée (i.e. abîmée) entre sa pointe et sa racine.

Or, la demanderesse vient maintenant de découvrir qu'il est possible d'obtenir de nouvelles teintures puissantes, peu sélectives et particulièrement résistantes, capables d'engendrer des colorations intenses dans des nuances variées, en utilisant des composés imidazoloazoles comme coupleurs en présence d'une base d'oxydation.

Cette découverte est à la base de la présente invention.

L'invention a pour objet une composition pour la teinture des fibres kératiniques et en particulier des fibres kératiniques humaines telles que les cheveux, caractérisée par le fait qu'elle comprend, dans un milieu approprié pour la teinture :
- à titre de coupleur, au moins un composé imidazoloazole de formule (I) ou l'un de ses sels d'addition avec un acide : 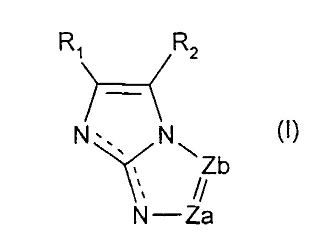 dans laquelle :
   . R₁ représente un atome d'hydrogène ; un atome d'halogène (tel que brome, chlore ou fluor) ; un radical alkyle en C₁-C₅, linéaire ou ramifié, éventuellement substitué par un ou deux radicaux halogène, hydroxy, alcoxy, aryloxy, amino, alkylamino, acyle, acylamino ; un radical alcoxy en C₁-C₄ ; un radical alkylthio en C₁-C₄ ; un radical arylthio ; un radical benzylthio, un radical acyle (tel que acétyle ; 3-phényl propanoyle, benzoyle ; 4-dodécyloxybenzoyle) ; un radical acylamino ; un radical acyloxy (tel que acétoxy) ; un radical carbamoyle (tel que carbamoyle ; N-éthylcarbamoyle, N-phénylcarbamoyle, N,N-dibutylcarbamoyle) ; N-(2-dodécyl-oxyéthyl) carbamoyle) ; un radical phényle éventuellement substitué par un ou deux groupes halogène, nitro, sulfonyle, alcoxy en C₁-C₄, alkyle en C₁-C₄, trifluoroalkyle en C₁-C₃, amino, alkylamino ; un radical alcoxy carbonyle (tel que méthoxycarbonyle, éthoxy-carbonyle, isopropyloxycarbonyle, tertiobutyloxycarbonyle, isobutyloxycarbonyle, butylcarbamoyléthoxycarbonyle, perfluorohexyléthoxycarbonyle ; un radical aryloxycarbonyle (tel que phénoxycarbonyle, 2,5-amyl phénoxycarbonyle) ; un radical cyano ; un radical nitro ; un radical dialkylphosphinyle (tel que diméthylphosphinyle) ; un radical alkylsulfinyle (tel que 3-phénoxypropyl sulfinyle) ; un radical arylsulfinyle (tel que phénylsulfinyle) ; un radical sulfamoyle (tel que N-éthylsulfamoyle, N,N-diisopropylsulfamoyle, N,N-diéthylsulfamoyle) ; un groupe carboxyle ; un groupe sulfo ; un radical aryloxy (tel que phénoxy, 2-méthylphénoxy, 4-tertio-butylphénoxy,3-nitrophénoxy) ; un radical alkylamino en C₁-C₄ ; uréido (tel que phényluréido, méthyluréido) ; sulfamoylamino (tel que N,N-dipropylsulfamoylamino) ; un radical alcoxycarbonylamino (tel que méthoxycarbonylamino, éthoxycarbonylamino); un radical sulfonamido (tel que méthane sulfonamido, benzène sulfonamido, toluylsulfonamido) ; un radical aryloxycarbonylamino (tel que phénoxycarbonylamino) ; un hétéroarylthio (tel que 2-benzothiazolylthio, 2-pyridylthio) un groupe phosphonyle (tel que phénylphosphonyle) ;
   . R₂ représente : un atome d'hydrogène ; un atome d'halogène tel que brome, chlore ou fluor ; un groupe acétylamido ; un radical alcoxy (tel que par exemple : méthoxy, éthoxy, propyloxy, benzyloxy, méthoxyéthoxy, phénoxyéthoxy, 2-cyanoéthoxy, phénéthyloxy, p-chlorobenzyloxy, méthoxyéthylcarbamoylméthoxy) ; un radical aryloxy (tel que par exemple : phénoxy, 4-méthoxyphénoxy, 4-nitrophénoxy, 4-cyano-phénoxy, 4-méthanesulfonamidophénoxy, 4-méthanesulfonylphénoxy, 3-méthylphénoxy, 1-naphtyloxy) ; un radical acyloxy (tel que par exemple : acétoxy, propanoyloxy, benzoyloxy, 2,4-dichlorobenzoyloxy, éthoxyalkyloxy, pyruviloyloxy, cinnamoyloxy, myristoyloxy) ; un radical arylthio (tel que par exemple : phénylthio, 4-carboxyphénylthio, 2-éthoxy 5-tert-butylphénylthio, 2-carboxyphénylthio, 4-méthane-sulfonylphénylthio) ; un radical alkylthio (tel que par exemple : méthylthio, éthylthio, propylthio, butylthio, 2-cyanoéthylthio, benzylthio, phénéthylthio, 2-(diéthylamino) éthylthio, éthoxyéthylthio, phénoxyéthylthio) ; un radical hétéroarylthio (tel que par exemple : 5-phényl 2,3,4,5-tétrazolylthio, 2-benzothiazolylthio) ; un radical hétéroaryloxy (tel que par exemple : 5-phényl 2,3,4,5-tétrazolyloxy, 2-benzo-thiazolyloxy) ; un radical thiocyano ; un radical N,N-diéthyl thiocarbonylthio ; un radical dodécyl-oxythio carbonylthio ; un radical benzènesulfonamido ; un radical N-éthyltoluène sulfonamido ; un radical pentafluorobutanamido ; un radical 2,3,4,5,6-pentafluorobenzamido ; un radical p-cyanophényluréido, un radical N,N-diéthyl-sulfamoylamino ; un radical pyrazolyle ; un radical imidazolyle ; un radical triazolyle ; un radical tétrazolyle ; un radical benzimidazolyle ; un radical 1-benzyl 5-éthoxy 3-hydantoïnyle ; un radical 1-benzyl 3-hydantoïnyle ; 5,5-diméthyl 2,4-dioxo 3-oxazolidinyle ; un radical 2-oxy 1,2-dihydro 1-pyridinyle ; un alkylamido ; un arylamido ; un radical NR^{III}R^{IV} avec R^{III} et R^{IV} représentant, identiques ou différents, un alkyle en C₁-C₄ , un hydroxyalkyle ; un carboxyle ; ou un radical alcoxycarboxylique.

   Zₐ, et Z_{b} représentent, indépendamment l'un de l'autre, un atome d'azote, un atome de carbone portant un radical R₃, R₄ ou R₅ sous réserve que l'un au moins des radicaux Zₐ et Z_{b} soit un atome de carbone.
   R₃ et R₄, indépendamment l'un de l'autre, représentent un atome d'hydrogène ; un atome d'halogène (tel que brome, chlore ou fluor) ; un radical alkyle en C₁-C₅, linéaire ou ramifié, éventuellement substitué par un ou deux radicaux halogène, hydroxy, alcoxy, aryloxy, amino, alkylamino, acyle, acylamino ; un radical arylthio ; un radical acyle (tel que acétyle ; 3-phényl propanoyle, benzoyle ; 4-dodécyloxybenzoyle) ; un radical acyloxy (tel que acétoxy) ; un radical carbamoyle (tel que carbamoyle ; N-éthylcarbamoyle) ; un radical phényle éventuellement substitué par un ou deux groupes halogène, nitro, sulfonyle, alcoxy en C₁-C₄, alkyle en C₁-C₄, trifluoroalkyle en C₁-C₃, amino, alkylamino ; un radical alcoxy carbonyle (tel que méthoxycarbonyle, éthoxy-carbonyle, isopropyloxycarbonyle, tertiobutyloxycarbonyle, isobutyloxy-carbonyle, butylcarbamoyléthoxycarbonyle, perfluorohexyléthoxycarbonyle ; un radical aryloxycarbonyle (tel que phénoxycarbonyle, 2,5-amyl phénoxycarbonyle) ; un radical cyano ; un radical nitro ; un radical dialkylphosphono (tel que diméthylphosphono) ; un radical diarylphosphono (tel que diphénylphosphono) ; un radical dialkylphosphinyle (tel que diméthylphosphinyle) ; un radical diarylphosphinyle (tel que diphénylphosphinyle) ; un radical alkylsulfinyle (tel que le 3-phénoxypropyl sulfinyle) ; un radical arylsulfinyle (tel que phénylsulfinyle) ; un radical arylsulfonyle (tel que le benzènesulfonyle, toluènesulfonyle) ; un radical alkylsulfonyle (méthanesulfonyle, octanesulfonyle) ; un radical sulfonyloxy (tel que méthanesulfonyloxy, toluènesulfonyloxy) ; un radical acylthio (tel que acétylthio, benzoylthio) ; un radical sulfamoyle (tel que N-éthylsulfamoyle, N,N-diisopropylsulfamoyle, N,N-diéthylsulfamoyle) ; un radical thiocyanate ; un radical thiocarbonyle (tel que méthylthiocarbonyle, phénylthio carbonyle) ; un radical alkylamino halogéné (tel que le N,N-di(trifluorométhylamino) ; un hétéroaryle (tel que 2-benzoxazolyle, 2-benzothiazolyle ; 5-chloro-1-tétrazolyle, 1-pyrolyle, 2-furyle, 2-thienyle).
   R₃ et R₄ peuvent former entre eux un cycle aromatique substitué ou non (tel que phényle) ;
   R₅ représente : un atome d'hydrogène ; un radical alkyle en C₁-C₂₀, linéaire ou ramifié, éventuellement substitué par 1 ou 2 radicaux R choisis dans le groupe constitué par halogène, nitro, cyano, hydroxy, alcoxy, aryloxy, amino, alkylamino, acylamino, carbamoyle, sulfonamido, sulfamoyle, imido, alkylthio, arylthio, aryle, alcoxycarbonyle, acyle ; un radical aryle (tel que phényle ou naphtyle), éventuellement substitué par 1 ou 2 radicaux R tels que précédemment définis ; un hétérocycle à 5 ou 6 chaînons possédant au moins un atome d'azote, d'oxygène ou de soufre (tel que pyridyle, quinolyle, pyrrolyle, morpholyle, furanyle, tétrahydrofuranyle, pyrazolyle, triazolyle, tétrazolyle, thiazolyle, oxazolyle, imidazolyle ou thiadiazolyle), éventuellement substitué par 1 ou 2 radicaux R tels que définis précédemment ;
   lorsque R₅ désigne un radical alkyle, un radical aryle ou un hétérocycle à 5 ou 6 chaînons (définis ci-dessus), il peut être relié à l'atome de carbone du noyau par l'intermédiaire d'un atome d'oxygène, d'azote ou de soufre (dans ce cas, R₅ devient XR₅ avec X = O, NH, S) ;
   R₅ peut désigner aussi un atome d'halogène (tel que brome, chlore ou fluor) ; un radical acyle ; un radical sulfonyle ; un radical sulfinyle ; un radical phosphonyle, un radical carbamoyle ; un radical sulfamoyle ; un radical cyano ; un radical siloxy, un radical amino ; un radical acylamino ; un radical acyloxy ; un radical carbamoyloxy ; un radical sulfonamide ; un radical imide ; un radical uréido ; un radical sulfamoylamino ; un radical alcoxycarbonylamino ; un radical aryloxycarbonylamino ; un radical alcoxycarbonyle ; un radical aryloxycarbonyle ; un radical carboxyle ;

   . et au moins une base d'oxydation.

   Les sels d'addition avec un acide des composés de l'invention peuvent être choisis notamment parmi des chlorhydrates, les bromhydrates, les tartrates, les tosylates, les benzènesulfonates, les sulfates, les lactates et les acétates.
   Parmi les radicaux R₁ de la formule (I) définie ci-dessus, on préfère les radicaux choisis dans le groupe constitué par :
   hydrogène, alkyle en C₁-C₄ ; phényle ; phényle substitué par un chlore, un brome, un nitro, un groupe sulfonyle, alcoxy en C₁-C₄, alkyle en C₁-C₄, un trifluoroalkyle en C₁-C₃, un amino ou un alkylamino en C₁-C₄ ; acyle ; acyloxy ; carbamoyle ; alcoxycarbonyle, aryloxycarbonyle ; cyano ; nitro ; alkylsulfinyle ; arylsulfinyle ; sulfamoyle ; alkyle halogéné.
   Parmi les radicaux R₁ de formule (I), on préfère plus particulièrement les radicaux choisis dans le groupe constitué par :
   hydrogène, alkyle en C₁-C₄ (tel que méthyle, éthyle, propyle, isopropyle, ter-butyle) ; phényle ; phényle substitué par un chlore, un sulfonyle, un méthoxy, un éthoxy, un alkyle en C₁-C₄ ou un groupe trifluorométhyle ; acyle (tel que acétyle, éthylcarbonyle, phénylcarbonyle) ; cyano ; alcoxycarbonyle (tel que méthoxycarbonyle, éthoxy-carbonyle) ; carbamoyle (tel que carbamoyle ; N-éthylcarbamoyle) ; trifluorométhyle.
   Et encore plus particulièrement, on préfère les radicaux R₁ choisis dans le groupe constitué par : hydrogène ; méthyle, éthyle ou isopropyle ; phényle ; phényle substitué par un chlore, un méthyle ou un méthoxy ; cyano ; méthoxycarbonyle ou éthoxy-carbonyle.
   Parmi les radicaux R₂ de la formule (I) définie ci-dessus, on préfère les radicaux choisis dans le groupe constitué par :
   un atome d'hydrogène ; un alcoxy en C₁-C₄ ; phénoxy ; phénoxy substitué par un atome d'halogène, un alkyle en C₁-C₄, un carboxyle, un groupe trifluorométhyle ; un radical acyloxy ; benzyloxy ; alkylthio en C₁-C₄ ; phénylthio ; phénylthio substitué par un atome d'halogène, un alkyle en C₁-C₄, un carboxyle, un groupe trifluorométhyle ; un alkylamido en C₁-C₄ ; phénylamido ; un radical NR^{III}R^{IV} avec R^{III} et R^{IV} représentant, identiques ou différents, un alkyle en C₁-C₄, un hydroxyalkyle en C₁-C₄ ; un carboxyle ; un radical alcoxycarboxylique en C₁-C₄.
   Parmi les radicaux R₂ de la formule (I) définie ci-dessus, on préfère plus particulièrement les radicaux choisis dans le groupe constitué par :
   hydrogène ; chlore ou brome ; méthoxy ou éthoxy ; phénoxy ; 4-méthylphénoxy ; acyloxy ; benzyloxy ; méthylthio ou éthylthio ; phénylthio ; 4-méthylphénylthio ; 2-tertio-butylphénylthio ; acétamido ; phénylacétamido ; diméthylamino ; diéthylamino ; éthyl-méthylamino ; (β-hydroxyéthyl)méthylamino.
   Et encore plus particulièrement, on préfère les radicaux R₂ choisis dans le groupe constitué par : hydrogène ; chlore ; éthoxy ; phénoxy ; benzyloxy ; acyloxy ; acétamido ; diméthylamino.
   Parmi les radicaux R₃ et R₄ de la formule (I), on préfère les radicaux choisis dans le groupe constitué par :
   acyle ; acyloxy ; carbamoyle ; alcoxycarbonyle ; aryloxycarbonyle ; cyano ; nitro; alkylsulfinyle ; arylsulfinyle ; alkylsulfonyle ; arylsulfonyle ; alkyle halogéné ; aryle ; hétéroaryle ; hydrogène ; alkyle en C₁-C₄ ; carboxyle ; hydrogène.
   Parmi les radicaux R₃ et R₄ de la formule (I), on préfère plus particulièrement les radicaux choisis dans le groupe constitué par : alcoxycarbonyle (tel que méthoxycarbonyle, éthoxycarbonyle) ; aryloxycarbonyle (tel que phénoxy carbonyle, chlorophénoxycarbonyle, toluyloxycarbonyle) ; nitro ; cyano ; arylsulfonyle (tel que phénylsulfonyle) ; carbamoyle (tel que carbamoyle ; N éthylcarbamoyle) ; alkyle halogéné (tel que trifluorométhyle) ; carboxyle ; hydrogène ; méthyle ; éthyle ou isopropyle ; hydrogène.
   Encore plus particulièrement, on préfère les radicaux R₃ et R₄ choisis dans le groupe constitué par :
   cyano ; trifluorométhyle, méthoxycarbonyle ; éthoxycarbonyle ; phénoxycarbonyle ; hydrogène ; méthyle ou éthyle ; carboxyle.
   Parmi les radicaux R₅ de la formule (I) définie ci-dessus, on préfère les radicaux choisis dans le groupe constitué par :
   un atome d'hydrogène ; un alkyle en C₁-C₄, linéaire ou ramifié ; un radical trifluorométhyle ; un phényle ; un phényle substitué par un ou deux groupes choisis parmi un halogène, un alkyle en C₁-C₄, un alcoxy en C₁-C₄, un hydroxy, un carboxyle, un groupe nitro, un alkylthio en C₁-C₄, un groupe méthylènedioxy, un groupe amino, un groupe trifluorométhyle ou un alkylamino en C₁-C₄ ; un radical benzyle ; un radical benzyle substitué par un atome d'halogène, un méthyle ou isopropyle, méthoxy ; un hydroxyalkyle en C₁-C₄ ; un aminoalkyle en C₁-C₄ ; un alkylaminoalkyle en C₁-C₄ ; un radical alcoxy choisi parmi méthoxy, éthoxy et phénoxy ; méthylthio ; éthylthio ; phénylthio ; méthanesulfonyle.
   Parmi les radicaux R₅ de la formule (I) définie ci-dessus, on préfère plus particulièrement les radicaux choisis dans le groupe constitué par : hydrogène ; un alkyle choisi parmi méthyle, éthyle, isopropyle, n-propyle, ter-butyle ; phényle ; toluyle ; 2-, 3- ou 4-chlorophényle ; 3- ou 4-hydroxyphényle ; 3- ou 4-aminophényle ; 3- ou 4-méthoxyphényle ; 4-trifluorométhylphényle ; benzyle ;
   trifluorométhyle ; hydroxyméthyle ; hydroxyéthyle ; hydroxyisopropyle ; aminométhyle ou aminoéthyle ; méthoxy ou éthoxy ; méthylthio ou éthylthio.

Et encore plus particulièrement, on préfère les radicaux R₅ choisis dans le groupe constitué par : hydrogène ; méthyle ; éthyle ; isopropyle ; phényle ; 4-chlorophényle ; 4-méthoxyphényle ; 4-aminophényle ; méthoxy ou éthoxy ; méthylthio ou éthylthio.

Parmi les composés de l'invention de formule (I) préférentiels, on peut citer ceux choisis dans le groupe constitué par :
(i) les imidazolo-[3,2-a] imidazoles de formule : 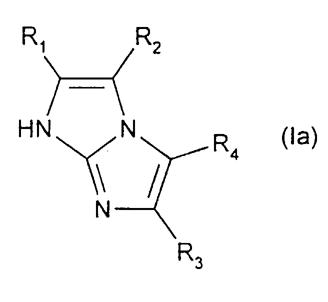
(ii) les imidazolo-[1,2-b]-1,2,4-triazoles de formule : 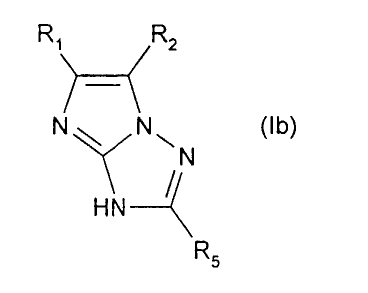
(iii) les imidazolo-[2,1-c]-1,2,4-triazoles de formule : 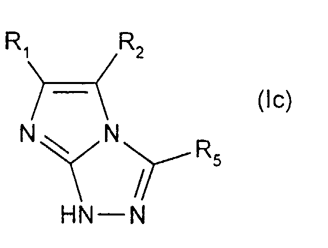 dans lesquelles R₁, R₂, R₃, R₄ et R₅ ont les mêmes significations que celles indiquées ci-dessus dans la formule (I).

Comme exemples de composés de formule (la), on peut citer ceux pour lesquels :
- R₁ désigne hydrogène, méthyle, éthyle, isopropyle ou phényle ;
- R₂ désigne hydrogène ou chlore ;
- R₃ et R₄ désignent respectivement cyano et cyano ; carboxyle et carboxyle ou méthoxycarbonyle et cyano.

A titre de composés de formule (Ia) ci-dessus, on peut tout particulièrement citer :
- le 7,8-dicyano-imidazolo- [3,2-a]- imidazole,
- le 7,8-dicyano-4-méthyl-imidazolo- [3,2-a]- imidazole,
- le 7,8-dicyano-4-éthyl-imidazolo- [3,2-a]- imidazole,
- le 7,8-dicyano-4-isopropyl-imidazolo- [3,2-a]- imidazole,
- le 7,8-dicyano-4-phényl-imidazolo- [3,2-a]- imidazole,
- le 5-chloro-7,8-dicyano-4-méthyl-imidazolo- [3,2-a]- imidazole,
- le 7,8-dicyano-4-trifluorométhyl-imidazolo- [3,2-a]- imidazole,
et leurs sels d'addition avec un acide.

Comme exemples de composés de formule (Ib), on peut citer ceux pour lesquels :
- R₁ désigne hydrogène, méthyle, éthyle, isopropyle ou phényle ;
- R₂ désigne hydrogène ou chlore ;
- R₅ désigne hydrogène, méthyle, éthyle, phényle, toluyle ou trifluorométhyle.

A titre de composés de formule (Ib) ci-dessus, on peut tout particulièrement citer :
- le imidazolo [1,2-b]-1,2,4-triazole,
- le 6-méthyl- imidazolo [1,2-b]-1,2,4-triazole,
- le 6-isopropyl- imidazolo [1,2-b]-1,2,4-triazole,
- le 6-phényl- imidazolo [1,2-b]-1,2,4-triazole,
- le 2,6-diméthyl- imidazolo [1,2-b]-1,2,4-triazole,
- le 6-isopropyl-2-méthyl- imidazolo [1,2-b]-1,2,4-triazole,
- le 2-méthyl-6-phényl- imidazolo [1,2-b]-1,2,4-triazole,
- le 6-méthyl-2-phényl- imidazolo [1,2-b]-1,2,4-triazole,
- le 6-isopropyl-2-phényl- imidazolo [1,2-b]-1,2,4-triazole,
- le 7-chloro-2,6-diméthyl- imidazolo [1,2-b]-1,2,4-triazole,
- le 7-chloro-2-phényl-6-tertbutyl- imidazolo [1,2-b]-1,2,4-triazole,
- le 6-trifluorométhyl- imidazolo [1,2-b]-1,2,4-triazole,
et leurs sels d'addition avec un acide.

Comme exemples de composés de formule (Ic), on peut citer ceux pour lesquels :
- R₁ désigne hydrogène, méthyle, éthyle, isopropyle ou phényle ;
- R₂ désigne hydrogène ou chlore ;
- R₅ désigne hydrogène, méthyle, éthyle, phényle, toluyle ou trifluorométhyle.

A titre de composés de formule (Ic) ci-dessus, on peut tout particulièrement citer :
- le imidazolo [2,1-c]-1, 2, 4-triazole,
- le 5-méthyl- imidazolo [2,1-c]- 1, 2, 4- triazole,
- le 5,8-diméthyl- imidazolo[2,1-c]-1, 2, 4-triazole,
- le 5-méthyl-8-phényl- imidazolo [2,1-c]- 1, 2, 4- triazole,
- le 8-phényl- imidazolo [2,1-c]-1, 2, 4- triazole,
- le 6-chloro-5,8-diméthyl- imidazolo [2,1-c]- 1, 2, 4- triazole,
et leurs sels d'addition avec un acide.

Les composés de la présente invention, leurs intermédiaires de synthèse et leurs procédés de préparation sont décrites dans les brevets et demandes de brevet US 5,441,863 ; JP 62-279 337 ; JP 06-236 011 et JP 07-092 632.

Le ou les composés de formule (I) représentent de préférence de 0,0005 à 12 % en poids environ du poids total de la composition tinctoriale, et encore plus préférentiellement de 0,005 à 6 % en poids environ de ce poids.

La nature de la ou des bases d'oxydation pouvant être utilisées dans la composition tinctoriale selon l'invention n'est pas critique. Cette ou ces bases d'oxydation sont de préférence choisies parmi les paraphénylènediamines, les bis-phénylalkylènediamines, les paraaminophénols, les ortho-aminophénols, les bases hétérocycliques, et leurs sels d'addition avec un acide.

Parmi les paraphénylènediamines utilisables à titre de bases d'oxydation dans la composition tinctoriale selon l'invention, on peut notamment citer les composés répondant à la formule (II) suivante, et leurs sels d'addition avec un acide : 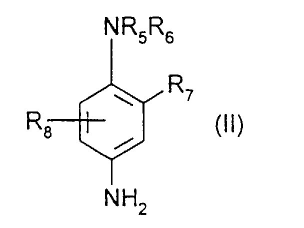 dans laquelle :
R₅ représente un atome d'hydrogène, un radical alkyle en C₁-C₄, monohydroxyalkyle en C₁-C₄, polyhydroxyalkyle en C₂-C₄ ou alcoxy(C₁-C₄)alkyle(C₁-C₄),
R₆ représente un atome d'hydrogène, un radical alkyle en C₁-C₄, monohydroxyalkyle en C₁-C₄ ou polyhydroxyalkyle en C₂-C₄,
R₇ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄,
R₈ représente un atome d'hydrogène ou un radical alkyle en C₁-C₄.

Dans la formule (Il) ci-dessus, et lorsque R₇ est différent d'un atome d'hydrogène, alors R₅ et R₈ représentent de préférence un atome d'hydrogène et R₇ est de préférence identique à R₈, et lorsque R₇ représente un atome d'halogène, alors R₅, R₆ et R₈ représentent de préférence un atome d'hydrogène.

Parmi les paraphénylènediamines de formule (II) ci-dessus, on peut plus particulièrement citer la paraphénylènediamine, la paratoluylènediamine, la 2-isopropyl paraphénylène-diamine, la 2-β-hydroxyéthyl paraphénylènediamine, la 2-β-hydroxyéthyloxy para-phénylènediamine, la 2,6-diméthyl paraphénylènediamine, la 2,6-diéthyl paraphénylène-diamine, la 2,3-diméthyl paraphénylènediamine, la N,N-bis-(β-hydroxyéthyl) para-phénylènediamine, le 4-amino 1-(β-méthoxyéthyl)amino benzène, la 2-chloro para-phénylènediamine, et leurs sels d'addition avec un acide.

Parmi les bis-phénylalkylènediamines utilisables à titre de bases d'oxydation dans la composition tinctoriale selon l'invention, on peut notamment citer les composés répondant à la formule (III) suivante, et leurs sels d'addition avec un acide : 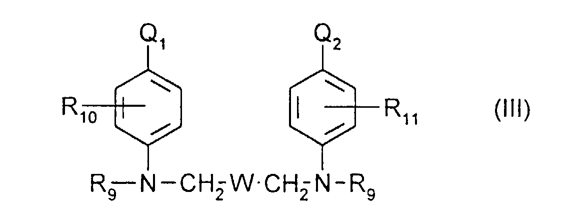 dans laquelle :
Q₁ et Q₂, identiques ou différents, représentent un radical hydroxyle ou NHR₁₂ dans lequel R₁₂ représente un atome d'hydrogène ou un radical alkyle en C₁-C₄, R₉ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₁₀ et R₁₁, identiques ou différents, représentent un atome d'hydrogène ou d'halogène ou un radical alkyle en C₁-C₄,
W représente un radical pris dans le groupe constitué par les radicaux suivants :
   -(CH₂)ₙ ; - (CH₂)ₘ-O-(CH₂)ₘ ; - (CH₂)ₘ-CHOH-(CH₂)ₘ et 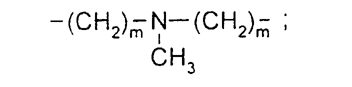 dans lesquels n est un nombre entier compris entre 0 et 8 inclusivement et m est un nombre entier compris entre 0 et 4 inclusivement.

Parmi les bis-phénylalkylènediamines de formule (III) ci-dessus, on peut plus particulièrement citer le N,N'-bis-(β-hydroxyéthyl) N,N'-bis-(4'-aminophényl) 1,3-diamino 2-propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (III), le N,N'-bis-(β-hydroxyéthyl) N,N'-bis-(4'-aminophényl) 1,3-diamino propanol ou l'un de ses sels d'addition avec un acide sont particulièrement préférés.

Parmi les paraaminophénols utilisables à titre de bases d'oxydation dans la composition tinctoriale selon l'invention, on peut notamment citer les composés répondant à la formule (IV) suivante, et leurs sels d'addition avec un acide : 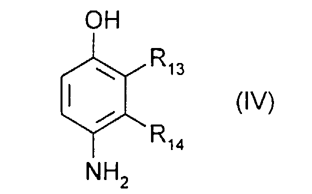 dans laquelle :
R₁₃ représente un atome d'hydrogène, un radical alkyle en C₁-C₄, monohydroxyalkyle en C₁-C₄, alcoxy(C₁-C₄)alkyle(C₁-C₄) ou aminoalkyle en C₁-C₄,
R₁₄ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₃ ou R₁₄ représente un atome d'hydrogène.

Parmi les paraaminophénols de formule (IV) ci-dessus, on peut plus particulièrement citer le paraaminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyethyl aminométhyl) phénol, et leurs sels d'addition avec un acide.

Parmi les ortho-aminophénols utilisables à titre de bases d'oxydation dans la composition tinctoriale selon l'invention, on peut notamment citer le 2-amino phénol, le 2-amino 1-hydroxy 5-méthyl benzène, le 2-amino 1-hydroxy 6-méthyl benzène, le 5-acétamido 2-amino phénol, et leurs sels d'addition avec un acide.

Parmi les bases hétérocycliques utilisables à titre de bases d'oxydation dans la composition tinctoriale selon l'invention, on peut notamment citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495, comme la 2,4,5,6-tétra-aminopyrimidine, la 4-hydroxy 2,5,6-triamino-pyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969 et WO 94/08970 comme le 4,5-diamino 1-méthyl pyrazole, le 3,4-diamino pyrazole, et leurs sels d'addition avec un acide et le 1-(4'-chlorobenzyl)-4,5-diaminopyrazole.

Selon l'invention, la ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale selon l'invention peut également renfermer un ou plusieurs coupleurs additionnels différents des composés de formule (I) et/ou un ou plusieurs colorants directs de façon à faire varier ou enrichir en reflets les nuances obtenues avec les bases d'oxydation.

Les coupleurs additionnels utilisables dans la composition selon l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, et leurs sels d'addition avec un acide.

Ces coupleurs peuvent notamment être choisis parmi le 2-méthyl 5-amino phénol, le 5-N-(β-hydroxyéthyl)amino 2-méthyl phénol, le 3-amino phénol, le 1,3-dihydroxybenzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'a-naphtol, le 6-hydroxy indole, le 4-hydroxy indole, le 4-hydroxy N-méthyl indole, la 6-hydroxy indoline, et leurs sels d'addition avec un acide.

Lorsqu'ils sont présents, ces coupleurs additionnels représentent de préférence de 0,0005 à 5 % en poids environ du poids total de la composition tinctoriale, et encore plus préférentiellement de 0,005 à 3 % en poids environ de ce poids.

Les sels d'addition avec un acide de la ou des bases d'oxydation et/ou des coupleurs additionnels utilisables dans la composition tinctoriale de l'invention sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcools inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 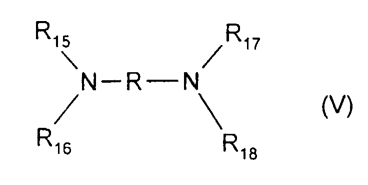 dans laquelle R est un reste propylène éventuellement substitué par un groupement hydroxyle ou un radical alkyle en C₁-C₄ ; R₁₅, R₁₆, R₁₇ et R₁₈, identiques ou différents, représentent un atome d'hydrogène, un radical alkyle en C₁-C₄ ou hydroxyalkyle en C₁-C₄.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet l'utilisation des imidazoloazoles de formule (I) ci-dessus, à titre de coupleur, en association avec au moins une base d'oxydation pour la teinture d'oxydation des fibres kératiniques et particulier des fibres kératiniques humaines telles que les cheveux.

Un autre objet de l'invention est un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus.

Ces dispositifs peuvent être équipés d'un moyen permettant de délivrer sur les cheveux le mélange souhaité, tel que les dispositifs décrits dans le brevet FR-2 586 913 au nom de la demanderesse.

### EXEMPLES

### EXEMPLE 1 DE TEINTURE

On a préparé la composition tinctoriale, conforme à l'invention, suivante :
- Paraphénylènediamine 0,324 g
- 7,8-dicyano-4-méthyl-imidazolo- [3,2-a]- imidazole (coupleur de formule (I) conforme à l'invention) 0,513 g
- Alcool benzylique 2,0 g
- Polyéthylèneglycol à 6 moles d'oxyde d'éthylène 3,0 g
- Alcool éthylique 20,0 g
- Alkyl (C₈-C₁₀) polyglucoside en solution aqueuse à 60% de matière active tamponné par du citrate d'ammonium, vendu sous la dénomination ORAMIX CG110 par la société SEPPIC 6,0 g
- Ammoniaque à 20 % de NH₃ 10,0 g
- Métabisulfite de sodium 0,228 g
- Agent séquestrant q.s.
- Eau déminéralisée q.s.p. 100,0 g
**NB :** le 7,8-dicyano-4-méthyl-imidazolo- [3,2-a]- imidazole a été préparé selon le procédé de synthèse décrit dans le brevet US 5 441 863.

Au moment de l'emploi, la composition tinctoriale ci-dessus définie a été mélangée avec un poids égal d'une solution de peroxyde d'hydrogène à 20 volumes (6% en poids).

Le mélange obtenu présentait un pH de 10,1 et a été appliqué pendant 30 minutes, sur des mèches de cheveux gris naturels à 90 % de blancs, permanentés ou non, à raison de 10 g pour 1 g de cheveux. Après rinçage, lavage avec un shampooing standard et séchage, les mèches ont été teintes dans les nuances figurant dans le tableau ci-dessous :

| **Exemple** | **Nuance obtenue sur cheveux gris naturels à 90% de blancs** | **Nuance obtenue sur cheveux gris à 90% de blancs permanentés** |
|---|---|---|
| **1** | Naturel légèrement doré | Naturel légèrement irisé |

### EXEMPLE 2 DE TEINTURE

On a préparé une composition tinctoriale identique à celle décrite pour l'exemple 1 ci-dessus.

Au moment de l'emploi, cette composition tinctoriale a été mélangée avec une quantité égale en poids d'une solution aqueuse de persulfate d'ammonium à 6.10⁻³ mole %.

Le mélange obtenu présentait un pH de 9,8 et a été appliqué pendant 30 minutes, sur des mèches de cheveux gris naturels à 90 % de blancs, à raison de 10 g pour 1 g de cheveux. Après rinçage, lavage avec un shampooing standard et séchage, les mèches ont été teintes dans une nuance dorée.

## Revendications

1. Composition pour la teinture des fibres kératiniques et en particulier des fibres kératiniques humaines telles que les cheveux, caractérisée par le fait qu'elle comprend dans un milieu approprié :
- à titre de coupleur, au moins un composé imidazoloazole de formule (I) suivante ou l'un de ses sels d'addition avec un acide : 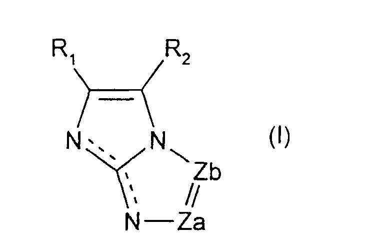 dans laquelle :
R₁ représente un atome d'hydrogène ; un atome d'halogène; un radical alkyle en C₁-C₅, linéaire ou ramifié, éventuellement substitué par un ou deux radicaux halogène, hydroxy, alcoxy, aryloxy, amino, alkylamino, acyle, acylamino ; un radical alcoxy en C₁-C₄ ; un radical alkylthio en C₁-C₄ ; un radical arylthio ; un radical benzylthio, un radical acyle; un radical acylamino ; un radical acyloxy ; un radical carbamoyle ; un radical phényle éventuellement substitué par un ou deux groupes halogène, nitro, sulfonyle, alcoxy en C₁-C₄, alkyle en C₁-C₄, trifluoroalkyle en C₁-C₃, amino, alkylamino ; un radical alcoxy carbonyle ; un radical aryloxycarbonyle ; un radical cyano ; un radical nitro ; un radical dialkylphosphinyle ; un radical arylsulfinyle ; un radical alkylsulfinyle ; un radical sulfamoyle ; un groupe carboxy ; un groupe sulfo ; un radical aryloxy ; un radical alkylamino en C₁-C₄ ; uréido ; sulfamoylamino ; un radical sulfonamido ; un radical alcoxycarbonylamino ; un radical aryloxycarbonylamino ; un hétéroarylthio ; un groupe phosphonyle ;
R₂ représente : un atome d'hydrogène ; un atome d'halogène ; un groupe acétylamido ; un radical alcoxy ; un radical aryloxy ; un radical acyloxy ; un radical arylthio ; un radical alkylthio ; un radical hétéroarylthio ; un radical hétéroaryloxy ; un radical thiocyano ; un radical N,N-diéthyl thiocarbonylthio ; un radical dodécyl-oxythio carbonylthio ; un radical benzènesulfonamido ; un radical N-éthyltoluènesulfonamido; un radical pentafluorobutanamido ; un radical 2,3,4,5,6-pentafluoro-benzamido ; un radical p-cyanophényluréido; un radical N,N-diéthyl-sulfamoylamino ; un radical pyrazolyle ; un radical imidazolyle ; un radical triazolyle ; un radical tétrazolyle ; un radical benzimidazolyle ; un radical 1-benzyl 5-éthoxy 3-hydantoïnyle ; un radical 1-benzyl 3-hydantoïnyle ; 5,5-diméthyl 2,4-dioxo 3-oxazolidinyle ; un radical 2-oxy 1,2-dihydro 1-pyridinyle ; un alkylamido ; un arylamido ; un radical NR^{III}R^{IV} avec R^{III} et R^{IV} représentant, identiques ou différents, un alkyle en C₁-C₄, un hydroxyalkyle ; un carboxyle ; ou un radical alcoxycarboxylique ;
Zₐ, et Z_{b} représentent, indépendamment l'un de l'autre, un atome d'azote, un atome de carbone portant un radical R₃, R₄ ou R₅ sous réserve que l'un au moins des radicaux Zₐ et Z_{b} soit un atome de carbone ;
R₃ et R₄, indépendamment l'un de l'autre, représentent un atome d'hydrogène ; un atome d'halogène ; un radical alkyle en C₁-C₅, linéaire ou ramifié, éventuellement substitué par un ou deux radicaux halogène, hydroxy, alcoxy, aryloxy, amino, alkylamino, acyle, acylamino ; un radical arylthio ; un radical acyle ; un radical acyloxy ; un radical carbamoyle ; un radical phényle éventuellement substitué par un ou deux groupes halogène, nitro, sulfonyle, alcoxy en C₁-C₄, alkyle en C₁-C₄, trifluoroalkyle en C₁-C₃, amino, alkylamino ; un radical alcoxycarbonyle ; un radical aryloxycarbonyle ; un radical cyano ; un radical nitro ; un radical dialkylphosphono ; un radical diarylphosphono ; un radical dialkylphosphinyle ; un radical diarylphosphinyle ; un radical alkylsulfinyle ; un radical arylsulfinyle ; un radical arylsulfonyle ; un radical alkylsulfonyle ; un radical sulfonyloxy ; un radical acylthio ; un radical sulfamoyle ; un radical thiocyanate ; un radical thiocarbonyle ; un radical alkylamino halogéné ; un hétérocycle ;
R₃ et R₄ peuvent former entre eux un cycle aromatique substitué ou non ;
R₅ représente : un atome d'hydrogène ; un radical alkyle en C₁-C₂₀, linéaire ou ramifié, éventuellement substitué par 1 ou 2 radicaux R choisis dans le groupe constitué par halogène, nitro, cyano, hydroxy, alcoxy, aryloxy, amino, alkylamino, acylamino, carbamoyle, sulfonamido, sulfamoyle, imido, alkylthio, arylthio, aryle, alcoxycarbonyle, acyle ; un radical aryle éventuellement substitué par 1 ou 2 radicaux R tels que précédemment définis ; un hétérocycle à 5 ou 6 chaînons possédant au moins un atome d'azote, d'oxygène ou de soufre, éventuellement substitué par 1 ou 2 radicaux R tels que définis précédemment ;
lorsque R₅ désigne un radical alkyle, un radical aryle ou un hétérocycle à 5 ou 6 chaînons (définis ci-dessus), il peut être relié à l'atome de carbone du noyau par l'intermédiaire d'un atome d'oxygène, d'azote ou de soufre (dans ce cas, R₅ devient XR₅ avec X = O, NH, S) ;
R₅ peut désigner aussi un atome d'halogène ; un radical acyle ; un radical sulfonyle ; un radical sulfinyle ; un radical phosphonyle, un radical carbamoyle ; un radical sulfamoyle ; un radical cyano ; un radical siloxy, un radical amino ; un radical acylamino ; un radical acyloxy ; un radical carbamoyloxy ; un radical sulfonamide ; un radical imide ; un radical uréido ; un radical sulfamoylamino ; un radical alcoxycarbonylamino ; un radical aryloxycarbonylamino ; un radical alcoxycarbonyle ; un radical aryloxycarbonyle ; un radical carboxyle ;
. et au moins une base d'oxydation.

2. Composition selon la revendication 1, caractérisée par le fait que les radicaux R₁ dans la formule (I) sont choisis dans le groupe constitué par : hydrogène ; alkyle en C₁-C₄ ; phényle ; phényle substitué par un chlore, un brome, un nitro, un sulfonyle, un alcoxy en C₁-C₄, un alkyle en C₁-C₄, un trifluoroalkyle en C₁-C₃ ; un amino ou un alkylamino en C₁-C₄ ; acyle ; acyloxy ; carbamoyle ; alcoxycarbonyle ; aryloxycarbonyle ; cyano ; nitro ; alkylsulfinyle ; arylsulfinyle ; sulfamoyle ; alkyle halogéné.

3. Composition selon la revendication 2, caractérisée par le fait que les radicaux R₁ dans la formule (I) sont choisis dans le groupe constitué par : hydrogène ; alkyle en C₁-C₄ ; phényle ; phényle substitué par un chlore, un sulfonyle, un méthoxy, un éthoxy, un alkyle en C₁-C₄ ou un trifluorométhyle ; acyle ; cyano ; alcoxycarbonyle ; carbamoyle ; trifluorométhyle.

4. Composition selon la revendication 2 ou 3, caractérisée par le fait que les radicaux R₁ sont choisis dans le groupe constitué par : hydrogène ; méthyle ; éthyle ; isopropyle ; phényle ; phényle substitué par un chlore, un méthyle ou un méthoxy ; cyano ; méthoxycarbonyle ; éthoxycarbonyle.

5. Composition selon l'une quelconque des revendications 1 à 4, caractérisée par le fait que les radicaux R₂ de la formule (I) sont choisis dans le groupe constitué par : un atome d'hydrogène ; un alcoxy en C₁-C₄ ; phénoxy ; phénoxy substitué par un atome d'halogène, un alkyle en C₁-C₄, un carboxyle, un groupe trifluorométhyle ; un radical acyloxy ; benzyloxy, alkylthio en C₁-C₄ ; phénylthio ; phénylthio substitué par un atome d'halogène, un alkyle en C₁-C₄, un carboxyle, un groupe trifluorométhyle ; un alkylamido en C₁-C₄ ; phénylamido ; un radical NR^{III}R^{IV} avec R^{III} et R^{IV} représentant, identiques ou différents, un alkyle en C₁-C₄, un hydroxyalkyle en C₁-C₄ ; un carboxyle ; un radical alcoxycarboxylique en C₁-C₄.

6. Composition selon la revendication 5, caractérisée par le fait que les radicaux R₂ de la formule (I) sont choisis dans le groupe constitué par : hydrogène ; chlore ou brome ; méthoxy ou éthoxy ; phénoxy ; 4-méthylphénoxy ; acyloxy ; benzyloxy ; méthylthio ou éthylthio ; phénylthio ; 4-méthylphénylthio ; 2-tertio-butylphénylthio; acétamido ; phénylacétamido ; diméthylamino ; diéthylamino ; éthylméthylamino ; (β-hydroxyéthyl) méthylamino.

7. Composition selon la revendication 6, caractérisée par le fait que les radicaux R₂ de la formule (I) sont choisis dans le groupe constitué par : hydrogène ; chlore ; éthoxy ; phénoxy ; benzyloxy ; acyloxy ; acétamido ; diméthylamino.

8. Composition selon l'une quelconque des revendications 1 à 7, caractérisée par le fait que les radicaux R₃ et R₄ de la formule (I) sont choisis dans le groupe constitué par : acyle ; acyloxy ; carbamoyle ; alcoxycarbonyle ; aryloxycarbonyle ; cyano ; nitro ; arylsulfinyle ; alkylsulfonyle ; arylsulfonyle ; alkyle halogéné ; aryle ; hétéroaryle ; alkyle en C₁-C₄ ; carboxyle ; hydrogène.

9. Composition selon la revendication 8, caractérisée par le fait que les radicaux R₃ et R₄ sont choisis dans le groupe constitué par : alcoxycarbonyle ; aryloxycarbonyle ; nitro ; cyano ; arylsulfonyle ; carbamoyle ; alkyle halogéné ; carboxyle ; méthyle ; éthyle ou isopropyle ; hydrogène.

10. Composition selon la revendication 9, caractérisée par le fait que les radicaux R₃ et R₄ sont choisis dans le groupe constitué par : cyano, trifluorométhyle, méthoxycarbonyle ; éthoxycarbonyle ; phénoxycarbonyle, hydrogène ; méthyle ; éthyle ou carboxyle.

11. Composition selon l'une quelconque des revendications 1 à 10, caractérisée par le fait que les radicaux R₅ de la formule (I) sont choisis dans le groupe constitué par :
un atome d'hydrogène ; un alkyle en C₁-C₄, linéaire ou ramifié ; un trifluorométhyle ; un phényle ; un phényle substitué par un ou deux groupes choisis parmi un halogène, un alkyle en C₁-C₄, un alcoxy en C₁-C₄, un hydroxy, un carboxyle, un groupe nitro, un alkylthio en C₁-C₄, un groupe méthylènedioxy, un groupe amino, un groupe trifluorométhyle ou un alkylamino en C₁-C₄ ; un radical benzyle ; un radical benzyle substitué par un atome d'halogène, un méthyle ou isopropyle, méthoxy ; un hydroxyalkyle en C₁-C₄ ; un aminoalkyle en C₁-C₄ ; un alkylaminoalkyle en C₁-C₄ ; un radical alcoxy choisi parmi méthoxy, éthoxy et phénoxy ; méthylthio ; éthylthio ; phénylthio ; méthanesulfonyle.

12. Composition selon la revendication 11, caractérisée par le fait que les radicaux R₅ de la formule (I) sont choisis dans le groupe constitué par :
hydrogène ; un alkyle choisi parmi méthyle, éthyle, isopropyle, n-propyle, tertiobutyle ; phényle ; toluyle ; 2-, 3- ou 4-chlorophényle ; 3- ou 4-hydroxyphényle ; 3- ou 4-aminophényle ; 3- ou 4-méthoxyphényle ; 4-trifluorométhylphényle ; benzyle ; trifluorométhyle ; hydroxyméthyle ; hydroxyéthyle ; hydroxyisopropyle ; aminométhyle ou aminoéthyle ; méthoxy ou éthoxy ; méthylthio ou éthylthio.

13. Composition selon la revendication 12, caractérisée par le fait que les radicaux R₅ de la formule (I) sont choisis dans le groupe constitué par :
hydrogène ; méthyle ; éthyle ; isopropyle ; phényle ; 4-chlorophényle ; 4-méthoxy-phényle ; 4-aminophényle ; méthoxy ou éthoxy ; méthylthio ou éthylthio.

14. Composition selon l'une quelconque des revendications 1 à 13, caractérisée par le fait que les composés de formule (I) sont choisis dans le groupe constitué par :
(i) les imidazolo-[3,2-a] imidazoles de formule : 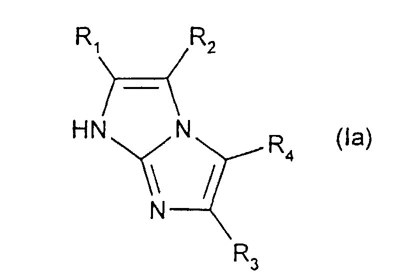
(ii) les imidazolo-[1,2-b]-1,2,4-triazoles de formule : 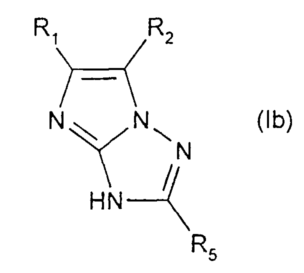
(iii) les imidazolo-[2,1-c]-1,2,4-triazoles de formule : 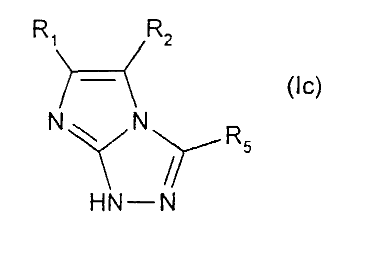 dans lesquelles R₁, R₂, R₃, R₄ et R₅ ont les mêmes significations que celles indiquées ci-dessus dans les revendications précédentes.

15. Composition selon la revendication 14, caractérisée par le fait que les composés de formule (Ia) sont choisis parmi ceux pour lesquels :
- R₁ désigne hydrogène, méthyle, éthyle, isopropyle ou phényle ;
- R₂ désigne hydrogène ou chlore ;
- R₃ et R₄ désignent respectivement cyano et cyano ; carboxyle et carboxyle ou méthoxycarbonyle et cyano.

16. Composition selon la revendication 14, caractérisée par le fait que les composés de formule (Ib) sont choisis dans le groupe constitué par ceux pour lesquels :
- R₁ désigne hydrogène, méthyle, éthyle, isopropyle ou phényle ;
- R₂ désigne hydrogène ou chlore ;
- R₅ désigne hydrogène, méthyle, éthyle, phényle, toluyle ou trifluorométhyle.

17. Composition selon la revendication 14, caractérisée par le fait que les composés de formule (Ic) sont choisis parmi ceux pour lesquels :
- R₁ désigne hydrogène, méthyle, éthyle, isopropyle ou phényle ;
- R₂ désigne hydrogène ou chlore ;
- R₅ désigne hydrogène, méthyle, éthyle, phényle, toluyle ou trifluorométhyle.

18. Composition selon la revendication 14, caractérisée par le fait que les composés de formule (I) sont choisis parmi :
- le 7,8-dicyano-imidazolo- [3,2-a]- imidazole,
- le 7,8-dicyano-4-méthyl-imidazolo- [3,2-a]- imidazole,
- le 7,8-dicyano-4-éthyl-imidazolo- [3,2-a]- imidazole,
- le 7,8-dicyano-4-isopropyl-imidazolo- [3,2-a]- imidazole,
- le 7,8-dicyano-4-phényl-imidazolo- [3,2-a]- imidazole,
- le 5-chloro-7,8-dicyano-4-méthyl-imidazolo- [3,2-a]- imidazole,
- le 7,8-dicyano-4-trifluorométhyl-imidazolo- [3,2-a]- imidazole,
- le imidazolo [1,2-b]-1,2,4-triazole,
- le 6-méthyl- imidazolo [1,2-b]-1,2,4-triazole,
- le 6-isopropyl- imidazolo [1,2-b]-1,2,4-triazole,
- le 6-phényl- imidazolo [1,2-b]-1,2,4-triazole,
- le 2,6-diméthyl- imidazolo [1,2-b]-1,2,4-triazole,
- le 6-isopropyl-2-méthyl- imidazolo [1,2-b]-1,2,4-triazole,
- le 2-méthyl-6-phényl- imidazolo [1,2-b]-1,2,4-triazole,
- le 6-méthyl-2-phényl- imidazolo [1,2-b]-1,2,4-triazole,
- le 6-isopropyl-2-phényl- imidazolo [1,2-b]-1,2,4-triazole,
- le 7-chloro-2,6-diméthyl- imidazolo [1,2-b]-1,2,4-triazole,
- le 7-chloro-2-phényl-6-tertbutyl- imidazolo [1,2-b]-1,2,4-triazole,
- le 6-trifluorométhyl- imidazolo [1,2-b]-1,2,4-triazole,
- le imidazolo [2,1-c]- 1, 2, 4- triazole,
- le 5-méthyl- imidazolo [2,1-c]- 1, 2, 4- triazole,
- le 5,8-diméthyl- imidazolo [2,1-c]- 1, 2, 4- triazole,
- le 5-méthyl-8-phényl- imidazolo [2,1-c]- 1, 2, 4- triazole,
- le 8-phényl- imidazolo [2,1-c]- 1, 2, 4- triazole,
- le 6-chloro-5,8-diméthyl- imidazolo [2,1-c]- 1, 2, 4- triazole,
et leurs sels d'addition avec un acide.

19. Composition selon l'une quelconque des revendications précédentes, caractérisée par le fait que les sels d'addition avec un acide des composés de formule (I) ou (II), sont choisis parmi les chlorhydrates, les bromhydrates, les tartrates, les tosylates, les benzènesulfonates, les sulfates, les lactates et les acétates.

20. Composition selon l'une quelconque des revendications précédentes, caractérisée par le fait que le ou les composés de formule (I) représentent de 0,0005 à 12 % en poids du poids total de la composition tinctoriale.

21. Composition selon la revendication 20, caractérisée par le fait que le ou les composés de formule (I) représentent de 0,005 à 6 % en poids du poids total de la composition tinctoriale.

22. Composition selon l'une quelconque des revendications précédentes, caractérisée par le fait que la ou les bases d'oxydation sont choisies parmi les paraphénylènediamines, les bis-phénylalkylènediamines, les paraaminophénols, les orthoaminophénols, les bases hétérocycliques, et leurs sels d'addition avec un acide.

23. Composition selon l'une quelconque des revendications précédentes, caractérisée par le fait que la ou les bases d'oxydation représentent de 0,0005 à 12 % en poids environ du poids total de la composition tinctoriale.

24. Composition selon la revendication 23, caractérisée par le fait que la ou les bases d'oxydation représentent de 0,005 à 6 % en poids environ du poids total de la composition tinctoriale.

25. Composition selon l'une quelconque des revendications précédentes, caractérisée par le fait qu'elle renferme en outre un ou plusieurs coupleurs additionnels différents des composés de formule (I) et/ou un ou plusieurs colorants directs.

26. Composition selon l'une quelconque des revendications précédentes, caractérisée par le fait que le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

27. Composition selon l'une quelconque des revendications précédentes, caractérisée par le fait qu'elle présente un pH compris entre 3 et 12.

28. Composition selon l'une quelconque des revendications précédentes, caractérisée par le fait qu'elle se présente sous forme de liquides, de crèmes, de gels, ou sous toute autre forme appropriée pour réaliser une teinture des fibres kératiniques, et notamment des cheveux humains.

29. Utilisation des composés de formule (I) ou de leurs sels d'addition avec un acide tels que définis à l'une quelconque des revendications 1 à 19, à titre de coupleurs dans des compositions pour la teinture d'oxydation des fibres kératiniques et en particulier des fibres kératiniques humaines telles que les cheveux, en association avec au moins une base d'oxydation.

30. Procédé de teinture d'oxydation des fibres kératiniques et en particulier des fibres kératiniques humaines telles que les cheveux, caractérisé par le fait que l'on applique sur ces fibres au moins une composition tinctoriale telle que définie à l'une quelconque des revendications 1 à 28, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

31. Procédé selon la revendication 30, caractérisé par le fait que l'agent oxydant est choisi parmi le peroxyde d'hydrogène, le peroxyde d'urée, les bromates de métaux alcalins, les persels tels que les perborates et persulfates.

32. Dispositif à plusieurs compartiments, ou "kit" de teinture à plusieurs compartiments, dont un premier compartiment renferme une composition tinctoriale telle que définie à l'une quelconque des revendications 1 à 28 et un second compartiment renferme une composition oxydante.

## Patentansprüche

1. Zusammensetzung zum Färben von Keratinfasern und insbesondere menschlichen Keratinfasern, wie dem Haar, dadurch gekennzeichnet, daß sie in einem zum Färben geeigneten Medium enthält:
- als Kuppler mindestens eine Imidazoloazol-Verbindung der folgenden Formel (I) oder ein Additionssalz dieser Verbindungen mit einer Säure: 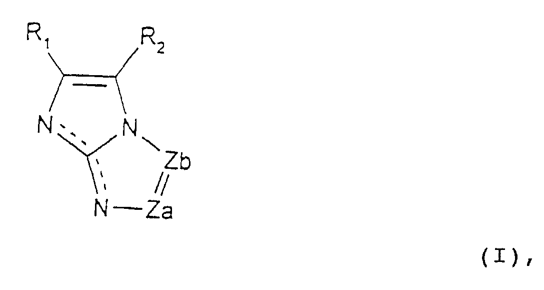 worin bedeuten:
- R₁ ein Wasserstoffatom, ein Halogenatom, eine geradkettige oder verzweigte C₁₋₅-Alkylgruppe, die gegebenenfalls mit einer oder zwei Halogen-, Hydroxy-, Alkoxy-, Aryloxy-, Amino-, Alkylamino-, Acyl- oder Acylaminogruppen substituiert ist, eine C₁₋₄-Alkoxygruppe, eine C₁₋₄-Alkylthiogruppe, eine Arylthiogruppe, eine Benzylthiogruppe, eine Acylgruppe, eine Acylaminogruppe, eine Acyloxygruppe, eine Carbamoylgruppe, eine Phenylgruppe, die gegebenenfalls mit einer oder zwei Halogen-, Nitro-, Sulfonyl-, C₁₋₄-Alkoxy-, C₁₋₄-Alkyl-, C₁₋₃-Trifluoralkyl-, Amino-oder Alkylaminogruppen substituiert ist, eine Alkoxycarbonylgruppe, eine Aryloxycarbonylgruppe, eine Cyanogruppe, eine Nitrogruppe, eine Dialkylphosphinylgruppe, eine Arylsulfinylgruppe, eine Alkylsulfinylgruppe, eine Sulfamoylgruppe, eine Carboxygruppe, eine Sulfogruppe, eine Aryloxygruppe, eine C₁₋₄-Alkylaminogruppe, eine Ureidogruppe, eine Sulfamoylaminogruppe, eine Sulfonamidogruppe, eine Alkoxycarbonylaminogruppe, eine Aryloxycarbonylaminogruppe, eine Heteroarylthiogruppe oder eine Phosphonylgruppe,
- R₂ ein Wasserstoffatom, ein Halogenatom, eine Acetylamidogruppe, eine Alkoxygruppe, eine Aryloxygruppe, eine Acyloxygruppe, eine Arylthiogruppe, eine Alkylthiogruppe, eine Heteroarylthiogruppe, eine Heteroaryloxygruppe, eine Thiocyanogruppe, eine N,N-Diethylthiocarbonylthiogruppe, eine Dodecyloxythiocarbonylthiogruppe, eine Benzolsulfonamidogruppe, eine N-Ethyltoluolsulfonamidogruppe, eine Pentafluorbutanamidogruppe, eine 2,3,4,5,6-Pentafluorbenzamidogruppe, eine p-Cyanophenylureidogruppe, eine N,N-Diethylsulfamoylaminogruppe, eine Pyrazolylgruppe, eine Imidazolylgruppe, eine Triazolylgruppe, eine Tetrazolylgruppe, eine Benzimidazolylgruppe, eine 1-Benzyl-5-ethoxy-3-hydantoinylgruppe, eine 1-Benzyl-3-hydantoinylgruppe, eine 5,5-Dimethyl-2,4-dioxo-3-oxazolidinylgruppe, eine 2-Oxy-1,2-dihydro-1-pyridinylgruppe, eine Alkylamidogruppe, eine Arylamidogruppe, eine Gruppe NR^{III}R^{IV}, wobei R^{III} und R^{IV}, die identisch oder voneinander verschieden sind, eine C₁₋₄-Alkylgruppe oder eine Hydroxyalkylgruppe bedeuten, eine Carboxygruppe oder eine Alkoxycarboxygruppe, und
- Zₐ und Z_{b} unabhängig voneinander ein Stickstoffatom oder ein Kohlenstoffatom, das eine Gruppe R₃, R₄ oder R₅ aufweist, mit der Maßgabe, daß mindestens eine der Gruppen Zₐ und Z_{b} ein Kohlenstoffatom bedeutet,
wobei:
- R₃ und R₄ unabhängig voneinander ein Wasserstoffatom, ein Halogenatom, eine geradkettige oder verzweigte C₁₋₅-Alkylgruppe, die gegebenenfalls mit einer oder zwei Halogen-, Hydroxy-, Alkoxy-, Aryloxy-, Amino-, Alkylamino-, Acyl- oder Acylaminogruppen substituiert ist, eine Arylthiogruppe, eine Acylgruppe, eine Acyloxygruppe, eine Carbamoylgruppe, eine Phenylgruppe, die gegebenenfalls mit einer oder zwei Halogen-, Nitro-, Sulfonyl-, C₁₋₄-Alkoxy-, C₁₋₄-Alkyl-, C₁₋₃-Trifluoralkyl-, Amino- oder Alkylaminogruppen substituiert ist, eine Alkoxycarbonylgruppe, eine Aryloxycarbonylgruppe, eine Cyanogruppe, eine Nitrogruppe, eine Dialkylphosphonogruppe, eine Diarylphosphonogruppe, eine Dialkylphosphinylgruppe, eine Diarylphosphinylgruppe, eine Alkylsulfinylgruppe, eine Arylsulfinylgruppe, eine Arylsulfonylgruppe, eine Alkylsulfonylgruppe, eine Sulfonyloxygruppe, eine Acylthiogruppe, eine Sulfamoylgruppe, eine Thiocyanatgruppe, eine Thiocarbonylgruppe, eine halogenierte Alkylaminogruppe oder einen Heterocyclus bedeuten, wobei R₃ und R₄ miteinander einen gegebenenfalls substituierten aromatischen Ring bilden können,
- R₅ ein Wasserstoffatom, eine geradkettige oder verzweigte C₁₋₂₀-Alkylgruppe, die gegebenenfalls mit einer oder zwei Gruppen R substituiert ist, die ausgewählt sind unter Halogen, Nitro, Cyano, Hydroxy, Alkoxy, Aryloxy, Amino, Alkylamino, Acylamino, Carbamoyl, Sulfonamido, Sulfamoyl, Imido, Alkylthio, Arylthio, Aryl, Alkoxycarbonyl und Acyl, eine Arylgruppe, die gegebenenfalls mit einer oder zwei wie oben definierten Gruppen R substituiert ist, einen Heterocyclus mit 5 oder 6 Gliedern, der mindestens ein Stickstoffatom, ein Sauerstoffatom oder ein Schwefelatom aufweist und gegebenenfalls mit einer oder zwei wie oben definierten Gruppen R substituiert ist, bedeutet, wobei die Gruppe R₅, wenn sie eine Alkylgruppe, eine Arylgruppe oder einen Heterocyclus mit 5 oder 6 Gliedern (die oben definiert sind) bedeutet, über ein Sauerstoffatom, ein Stickstoffatom oder ein Schwefelatom an das Kohlenstoffatom des Rings gebunden sein kann (R₅ wird in diesem Fall XR₅ mit X = O, NH, S), und
- R₅ ferner ein Halogenatom, eine Acylgruppe, eine Sulfonylgruppe, eine Sulfinylgruppe, eine Phosphonylgruppe, eine Carbamoylgruppe, eine Sulfamoylgruppe, eine Cyanogruppe, eine Siloxygruppe, eine Aminogruppe, eine Acylaminogruppe, eine Acyloxygruppe, eine Carbamoyloxygruppe, eine Sulfonamidgruppe, eine Imidgruppe, eine Ureidogruppe, eine Sulfamoylaminogruppe, eine Alkoxycarbonylaminogruppe, eine Aryloxycarbonylaminogruppe, eine Alkoxycarbonylgruppe, eine Aryloxycarbonylgruppe oder eine Carboxygruppe bedeuten kann,
- und mindestens eine Oxidationsbase.

2. Zusammensetzung nach Anspruch 1, dadurch gekennzeichnet, daß die Gruppen R₁ in der Formel (I) ausgewählt sind unter: Wasserstoff, C₁₋₄-Alkyl, Phenyl, Phenyl, das mit einem Chloratom, einem Bromatom, einer Nitrogruppe, einer Sulfonylgruppe, einer C₁₋₄-Alkoxygruppe, einer C₁₋₄-Alkylgruppe, einer C₁₋₃-Trifluoralkylgruppe, einer Aminogruppe oder einer C₁₋₄-Alkylaminogruppe substituiert ist, Acyl, Acyloxy, Carbamoyl, Alkoxycarbonyl, Aryloxycarbonyl, Cyano, Nitro, Alkylsulfinyl, Arylsulfinyl, Sulfamoyl und halogeniertem Alkyl.

3. Zusammensetzung nach Anspruch 2, dadurch gekennzeichnet, daß die Gruppen R₁ in der Formel (I) ausgewählt sind unter: Wasserstoff, C₁₋₄-Alkyl, Phenyl, Phenyl, das mit einem Chloratom, einer Sulfonylgruppe, einer Methoxygruppe, einer Ethoxygruppe, einer C₁₋₄-Alkylgruppe oder einer Trifluormethylgruppe substituiert ist, Acyl, Cyano, Alkoxycarbonyl, Carbamoyl und Trifluormethyl.

4. Zusammensetzung nach Anspruch 2 oder 3, dadurch gekennzeichnet, daß die Gruppen R₁ ausgewählt sind unter: Wasserstoff, Methyl, Ethyl, Isopropyl, Phenyl, Phenyl, das mit einem Chloratom, einer Methylgruppe oder einer Methoxygruppe substituiert ist, Cyano, Methoxycarbonyl und Ethoxycarbonyl.

5. Zusammensetzung nach einem der Ansprüche 1 bis 4, dadurch gekennzeichnet, daß die Gruppen R₂ der Formel (I) ausgewählt sind unter: Wasserstoff, C₁₋₄-Alkoxy, Phenoxy, Phenoxy, das mit einem Halogenatom, einer C₁₋₄-Alkylgruppe, einer Carboxygruppe oder einer Trifluormethylgruppe substituiert ist, Acyloxy, Benzyloxy, C₁₋₄-Alkylthio, Phenylthio, Phenylthio, das mit einem Halogenatom, einer C₁₋₄-Alkylgruppe, einer Carboxygruppe oder einer Trifluormethylgruppe substituiert ist, C₁₋₄-Alkylamido, Phenylamido, einer Gruppe NR^{III}R^{IV}, wobei R^{III} und R^{IV}, die identisch oder voneinander verschieden sind, C₁₋₄-Alkyl oder C₁₋₄-Hydroxyalkyl bedeuten, Carboxy und C₁₋₄-Alkoxycarboxy.

6. Zusammensetzung nach Anspruch 5, dadurch gekennzeichnet, daß die Gruppen R₂ der Formel (I) ausgewählt sind unter: Wasserstoff, Chlor oder Brom, Methoxy oder Ethoxy, Phenoxy, 4-Methylphenoxy, Acyloxy, Benzyloxy, Methylthio oder Ethylthio, Phenylthio, 4-Methylphenylthio, 2-tert.-Butylphenylthio, Acetamido, Phenylacetamido, Dimethylamino, Diethylamino, Ethylmethylamino und (β-Hydroxyethyl)methylamino.

7. Zusammensetzung nach Anspruch 6, dadurch gekennzeichnet, daß die Gruppen R₂ der Formel (I) ausgewählt sind unter: Wasserstoff, Chlor, Ethoxy, Phenoxy, Benzyloxy, Acyloxy, Acetamido und Dimethylamino.

8. Zusammensetzung nach einem der Ansprüche 1 bis 7, dadurch gekennzeichnet, daß die Gruppen R₃ und R₄ der Formel (I) ausgewählt sind unter: Acyl, Acyloxy, Carbamoyl, Alkoxycarbonyl, Aryloxycarbonyl, Cyano, Nitro, Arylsulfinyl, Alkylsulfonyl, Arylsulfonyl, halogeniertem Alkyl, Aryl, Heteroaryl, C₁₋₄-Alkyl, Carboxy und Wasserstoff.

9. Zusammensetzung nach Anspruch 8, dadurch gekennzeichnet, daß die Gruppen R₃ und R₄ ausgewählt sind unter: Alkoxycarbonyl, Aryloxycarbonyl, Nitro, Cyano, Arylsulfonyl, Carbamoyl, halogeniertem Alkyl, Carboxy, Methyl, Ethyl oder Isopropyl und Wasserstoff.

10. Zusammensetzung nach Anspruch 9, dadurch gekennzeichnet, daß die Gruppen R₃ und R₄ ausgewählt sind unter: Cyano, Trifluormethyl, Methoxycarbonyl, Ethoxycarbonyl, Phenoxycarbonyl, Wasserstoff, Methyl, Ethyl oder Carboxy.

11. Zusammensetzung nach einem der Ansprüche 1 bis 10, dadurch gekennzeichnet, daß die Gruppen R₅ der Formel (I) ausgewählt sind unter: Wasserstoff, geradkettigem oder verzweigtem C₁₋₄-Alkyl, Trifluormethyl, Phenyl, Phenyl, das mit einer oder zwei Gruppen substituiert ist, die ausgewählt sind unter Halogen, C₁₋₄-Alkyl, C₁₋₄-Alkoxy, Hydroxy, Carboxy, Nitro, C₁₋₄-Alkylthio, Methylendioxy, Amino, Trifluormethyl oder C₁₋₄-Alkylamino, Benzyl, Benzyl, das mit einem Halogenatom, Methyl, Isopropyl oder Methoxy substituiert ist, C₁₋₄-Hydroxyalkyl, C₁₋₄-Aminoalkyl, C₁₋₄-Alkylaminoalkyl, Alkoxy, das unter Methoxy, Ethoxy und Phenoxy ausgewählt ist, Methylthio, Ethylthio, Phenylthio und Methansulfonyl.

12. Zusammensetzung nach Anspruch 11, dadurch gekennzeichnet, daß die Gruppen R₅ der Formel (I) ausgewählt sind unter: Wasserstoff, Alkyl, das unter Methyl, Ethyl, Isopropyl, n-Propyl und *tert*.-Butyl ausgewählt ist, Phenyl, Toluyl, 2-, 3- oder 4-Chlorphenyl, 3- oder 4-Hydroxyphenyl, 3- oder 4-Aminophenyl, 3- oder 4-Methoxyphenyl, 4-Trifluormethylphenyl, Benzyl, Trifluormethyl, Hydroxymethyl, Hydroxyethyl, Hydroxyisopropyl, Aminomethyl oder Aminoethyl, Methoxy oder Ethoxy und Methylthio oder Ethylthio.

13. Zusammensetzung nach Anspruch 12, dadurch gekennzeichnet, daß die Gruppen R₅ der Formel (I) ausgewählt sind unter: Wasserstoff, Methyl, Ethyl, Isopropyl, Phenyl, 4-Chlorphenyl, 4-Methoxyphenyl, 4-Aminophenyl, Methoxy oder Ethoxy und Methylthio oder Ethylthio.

14. Zusammensetzung nach einem der Ansprüche 1 bis 13, dadurch gekennzeichnet, daß die Verbindungen der Formel (I) ausgewählt sind unter:
(i) den Imidazolo[3,2-a]imidazolen der Formel: 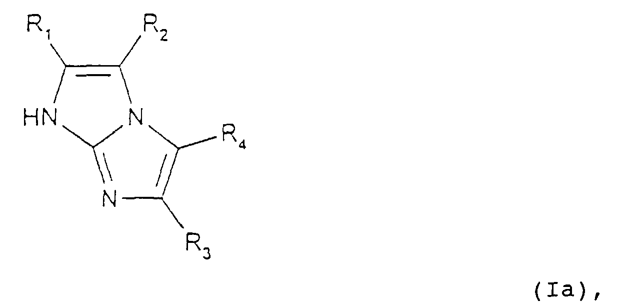
(ii) den Imidazolo[1,2-b]1,2,4-triazolen der Formel: 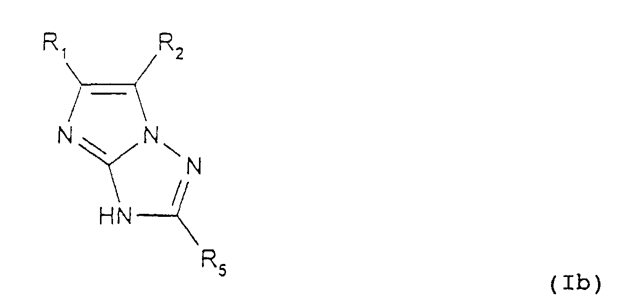 und
(iii) den Imidazolo[2,1-c]1,2,4-triazolen der Formel: 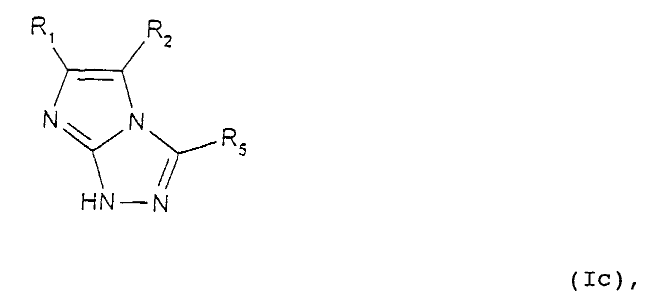 worin R₁, R₂, R₃, R₄ und R₅ die oben in den vorhergehenden Ansprüchen angegebenen Bedeutungen aufweisen.

15. Zusammensetzung nach Anspruch 14, dadurch gekennzeichnet, daß die Verbindungen der Formel (Ia) unter den Verbindungen ausgewählt sind, worin:
- R₁ Wasserstoff, Methyl, Ethyl, Isopropyl oder Phenyl bedeutet,
- R₂ Wasserstoff oder Chlor bedeutet und
- R₃ und R₄ Cyano und Cyano, Carboxy und Carboxy bzw. Methoxycarbonyl und Cyano bedeuten.

16. Zusammensetzung nach Anspruch 14, dadurch gekennzeichnet, daß die Verbindungen der Formel (Ib) unter den Verbindungen ausgewählt sind, worin:
- R₁ Wasserstoff, Methyl, Ethyl, Isopropyl oder Phenyl bedeutet,
- R₂ Wasserstoff oder Chlor bedeutet und
- R₅ Wasserstoff, Methyl, Ethyl, Phenyl, Toluyl oder Trifluormethyl bedeutet.

17. Zusammensetzung nach Anspruch 14 dadurch gekennzeichnet, daß die Verbindungen der Formel (Ic) unter den Verbindungen ausgewählt sind, worin:
- R₁ Wasserstoff, Methyl, Ethyl, Isopropyl oder Phenyl bedeutet,
- R₂ Wasserstoff oder Chlor bedeutet und
- R₅ Wasserstoff, Methyl, Ethyl, Phenyl, Toluyl oder Trifluormethyl bedeutet.

18. Zusammensetzung nach Anspruch 14, dadurch gekennzeichnet, daß die Verbindungen der Formel (I) ausgewählt sind unter:
- 7,8-Dicyano-imidazolo[3,2-a]imidazol,
- 7,8-Dicyano-4-methyl-imidazolo[3,2-a]imidazol,
- 7,8-Dicyano-4-ethyl-imidazolo[3,2-a]imidazol,
- 7,8-Dicyano-4-isopropyl-imidazolo[3,2-a]imidazol,
- 7,8-Dicyano-4-phenyl-imidazolo[3,2-a]imidazol,
- 5-Chlor-7,8-dicyano-4-methyl-imidazolo[3,2-a]imidazol,
- 7,8-Dicyano-4-trifluormethyl-imidazolo[3,2-a]imidazol,
- Imidazolo[1,2-b]1,2,4-triazol,
- 6-Methyl-imidazolo[1,2-b]1,2,4-triazol,
- 6-Isopropyl-imidazolo[1,2-b]1,2,4-triazol,
- 6-Phenyl-imidazolo[1,2-b]1,2,4-triazol,
- 2,6-Dimethyl-imidazolo[1,2-b]1,2,4-triazol,
- 6-Isopropyl-2-methyl-imidazolo[1,2-b]1,2,4-triazol,
- 2-Methyl-6-phenyl-imidazolo[1,2-b]1,2,4-triazol,
- 6-Methyl-2-phenyl-imidazolo[1,2-b]1,2,4-triazol,
- 6-Isopropyl-2-phenyl-imidazolo[1,2-b]1,2,4-triazol,
- 7-Chlor-2,6-dimethyl-imidazolo[1,2-b]1,2,4-triazol,
- 7-Chlor-2-phenyl-6-tert.-butyl-imidazolo[1,2-b]1,2,4-triazol,
- 6-Trifluormethyl-imidazolo[1,2-b]1,2,4-triazol,
- Imidazolo[2,1-c]1,2,4-triazol,
- 5-Methyl-imidazolo[2,1-c]1,2,4-triazol,
- 5,8-Dimethyl-imidazolo[2,1-c]1,2,4-triazol,
- 5-Methyl-8-phenyl-imidazolo[2,1-c]1,2,4-triazol,
- 8-Phenyl-imidazolo[2,1-c]1,2,4-triazol,
- 6-Chlor-5,8-dimethyl-imidazolo[2,1-c]1,2,4-triazol,
und den Additionssalzen dieser Verbindungen mit einer Säure.

19. Zusammensetzung nach einem der vorhergehenden Ansprüche, dadurch gekennzeichnet, daß die Additionssalze der Verbindungen der Formel (I) mit einer Säure unter Hydrochloriden, Hydrobromiden, Tartraten, Tosylaten, Benzolsulfonaten, Sulfaten, Lactaten und Acetaten ausgewählt sind.

20. Zusammensetzung nach einem der vorhergehenden Ansprüche, dadurch gekennzeichnet, daß die Verbindung oder die Verbindungen der Formel (I) 0,0005 bis 12 Gew.-% des Gesamtgewichts der Färbemittelzusammensetzung ausmachen.

21. Zusammensetzung nach Anspruch 20, dadurch gekennzeichnet, daß die Verbinding oder die Verbindungen der Formel (I) 0,005 bis 6 Gew.-% des Gesamtgewichts der Färbemittelzusammensetzung ausmachen.

22. Zusammensetzung nach einem der vorhergehenden Ansprüche, dadurch gekennzeichnet, daß die Oxidationsbase oder die Oxidationsbasen unter p-Phenylendiaminen, Bisphenylalkylendiaminen, p-Aminophenolen, o-Aminophenolen, heterocyclischen Basen und den Additionssalzen dieser Verbindungen mit einer Säure ausgewählt sind.

23. Zusammensetzung nach einem der vorhergehenden Ansprüche, dadurch gekennzeichnet, daß die Oxidationsbase oder die Oxidationsbasen etwa 0,0005 bis 12 Gew.-% des Gesamtgewichts der Färbemittelzusammensetzung ausmachen.

24. Zusammensetzung nach Anspruch 23, dadurch gekennzeichnet, daß die Oxidationsbase oder die Oxidationsbasen etwa 0,005 bis 6 Gew.-% des Gesamtgewichts der Färbemittelzusammensetzung ausmachen.

25. Zusammensetzung nach einem der vorhergehenden Ansprüche, dadurch gekennzeichnet, daß sie ferner einen oder mehrere zusätzliche Kuppler, die von den Verbindungen der Formel (I) verschieden sind, und/oder einen oder mehrere Direktfarbstoffe enthält.

26. Zusammensetzung nach einem der vorhergehenden Ansprüche, dadurch gekennzeichnet, daß das zum Färben geeignete Medium (oder der Träger) aus Wasser oder einem Gemisch von Wasser und mindestens einem organischen Lösemittel, das unter niederen Alkanolen mit 1 bis 4 Kohlenstoffatomen, Glycerin, Glykolen und Glykolethern, aromatischen Alkoholen, analogen Produkten und deren Gemischen ausgewählt ist, besteht.

27. Zusammensetzung nach einem der vorhergehenden Ansprüche, dadurch gekennzeichnet, daß sie einen pH-Wert im Bereich von 3 bis 12 aufweist.

28. Zusammensetzung nach einem der vorhergehenden Ansprüche, dadurch gekennzeichnet, daß sie als Flüssigkeit, Creme, Gel oder in beliebigen weiteren Formen vorliegt, die zur Durchführung einer Färbung von Keratinfasern und insbesondere des menschlichen Haares geeignet sind.

29. Verwendung der in einem der Ansprüche 1 bis 19 definierten Verbindungen der Formel (I) oder der Additionssalze dieser Verbindungen mit einer Säure als Kuppler in Kombination mit mindestens einer Oxidationsbase in Zusammensetzungen zum oxidativen Färben von Keratinfasern und insbesondere menschlichen Keratinfasern, wie dem Haar.

30. Verfahren zum oxidativen Färben von Keratinfasern und insbesondere menschlichen Keratinfasern, wie dem Haar, dadurch gekennzeichnet, daß mindestens eine in einem der Ansprüche 1 bis 28 definierte Färbemittelzusammensetzung auf die Fasern aufgebracht wird, wobei die Farbe bei saurem, neutralem oder alkalischem pH-Wert mit einem Oxidationsmittel entwickelt wird, das unmittelbar bei der Anwendung zu der Färbemittelzusammensetzung gegeben wird oder das in einer oxidierenden Zusammensetzung vorliegt, die gleichzeitig oder getrennt davon anschließend aufgebracht wird.

31. Verfahren nach Anspruch 30, dadurch gekennzeichnet, daß das Oxidationsmittel unter Wasserstoffperoxid, Harnstoffperoxid, Alkalimetallbromaten und Salzen von Persäuren, wie Perboraten und Persulfaten, ausgewählt ist.

32. Vorrichtung zum Färben oder 'Kit' mit mehreren Abteilungen, wobei eine erste Abteilung eine in einem der Ansprüche 1 bis 28 definierte Färbemittelzusammensetzung und eine zweite Abteilung eine oxidierende Zusammensetzung enthält.

## Claims

1. Composition for dyeing keratin fibres, and in particular human keratin fibres such as the hair, characterized in that it comprises, in a suitable medium:
- as coupler, at least one imidazoloazole compound of formula (I) below, or one of the addition salts thereof with an acid: 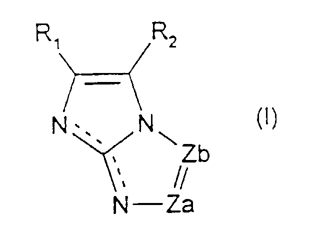 in which:
R₁ represents a hydrogen atom; a halogen atom; a linear or branched C₁-C₅ alkyl radical optionally substituted with one or two halogen, hydroxyl, alkoxy, aryloxy, amino, alkylamino, acyl or acylamino radicals; a C₁-C₄ alkoxy radical; a C₁-C₄ alkylthio radical; an arylthio radical; a benzylthio radical, an acyl radical; an acylamino radical; an acyloxy radical; a carbamoyl radical; a phenyl radical optionally substituted with one or two halogen, nitro, sulphonyl, C₁-C₄ alkoxy, C₁-C₄ alkyl, C₁-C₃ trifluoroalkyl, amino or alkylamino groups; an alkoxycarbonyl radical; an aryloxycarbonyl radical; a cyano radical; a nitro radical; a dialkylphosphinyl radical; an arylsulphinyl radical; an alkylsulphinyl radical; a sulphamoyl radical; a carboxyl group; a sulpho group; an aryloxy radical; a C₁-C₄ alkylamino radical; ureido; sulphamoylamino; a sulphonamido radical; an alkoxycarbonylamino radical; an aryloxycarbonylamino radical; a heteroarylthio; a phosphonyl group;
R₂ represents: a hydrogen atom; a halogen atom; an acetylamido group; an alkoxy radical; an aryloxy radical; an acyloxy radical; an arylthio radical; an alkylthio radical; a heteroarylthio radical; a heteroaryloxy radical; a thiocyano radical; an N,N-diethylthiocarbonylthio radical; a dodecyloxythiocarbonylthio radical; a benzenesulphonamido radical; an N-ethyltoluenesulphonamido radical; a pentafluorobutanamido radical; a 2,3,4,5,6-pentafluorobenzamido radical; a p-cyanophenylureido radical; an N,N-diethylsulphamoylamino radical; a pyrazolyl radical; an imidazolyl radical; a triazolyl radical; a tetrazolyl radical; a benzimidazolyl radical; a 1-benzyl-5-ethoxy-3-hydantoinyl radical; a 1-benzyl-3-hydantoinyl radical; 5,5-dimethyl-2,4-dioxo-3-oxazolidinyl; a 2-oxy-l,2-dihydro-l-pyridyl radical; an alkylamido; an arylamido; a radical NR^{III}R^{IV} with R^{III} and R^{IV}, which may be identical or different, representing a C₁-C₄ alkyl, a hydroxyalkyl; a carboxyl; or an alkoxycarboxylic radical;
Zₐ and Z_{b} represent, independently of each other, a nitrogen atom or a carbon atom bearing a radical R₃, R₄ or R₅, with the proviso that at least one of the radicals Zₐ and Z_{b} is a carbon atom;
R₃ and R₄ represent, independently of each other, a hydrogen atom; a halogen atom; a linear or branched C₁-C₅ alkyl radical optionally substituted with one or two halogen, hydroxyl, alkoxy, aryloxy, amino, alkylamino, acyl or acylamino radicals; an arylthio radical; an acyl radical; an acyloxy radical; a carbamoyl radical; a phenyl radical optionally substituted with one or two halogen, nitro, sulphonyl, C₁-C₄ alkoxy, C₁-C₄ alkyl, C₁-C₃ trifluoroalkyl, amino or alkylamino groups; an alkoxycarbonyl radical; an aryloxycarbonyl radical; a cyano radical; a nitro radical; a dialkylphosphono radical; a diarylphosphono radical; a dialkylphosphinyl radical; a diarylphosphinyl radical; an alkylsulphinyl radical; an arylsulphinyl radical; an arylsulphonyl radical; an alkylsulphonyl radical; a sulphonyloxy radical; an acylthio radical; a sulphamoyl radical; a thiocyanate radical; a thiocarbonyl radical; a haloalkylamino radical; a heterocycle;
R₃ and R₄ can together form a substituted or unsubstituted aromatic ring;
R₅ represents: a hydrogen atom; a linear or branched C₁-C₂₀ alkyl radical optionally substituted with one or two radicals R chosen from the group consisting of halogen, nitro, cyano, hydroxyl, alkoxy, aryloxy, amino, alkylamino, acylamino, carbamoyl, sulphonamido, sulphamoyl, imido, alkylthio, arylthio, aryl, alkoxycarbonyl, acyl; an aryl radical optionally substituted with one or two radicals R as defined above; a 5- or 6-membered heterocycle having at least one nitrogen, oxygen or sulphur atom optionally substituted with one or two radicals R as defined above;
when R₅ denotes an alkyl radical, an aryl radical or a 5- or 6-membered heterocycle (defined above), it can be linked to the carbon atom of the ring via an oxygen, nitrogen or sulphur atom (in this case, R₅ becomes XR₅ with X = O, NH, S);
R₅ can also denote a halogen atom; an acyl radical; a sulphonyl radical; a sulphinyl radical; a phosphonyl radical, a carbamoyl radical; a sulphamoyl radical; a cyano radical; a siloxy radical, an amino radical; an acylamino radical; an acyloxy radical; a carbamoyloxy radical; a sulphonamide radical; an imide radical; a ureido radical; a sulphamoylamino radical; an alkoxycarbonylamino -radical; an aryloxycarbonylamino radical; an alkoxycarbonyl radical; an aryloxycarbonyl radical; a carboxyl radical;
and at least one oxidation base.

2. Composition according to Claim 1, characterized in that the radicals R₁ in formula (I) are chosen from the group consisting of: hydrogen, C₁-C₄ alkyl; phenyl; phenyl substituted with a chlorine, a bromine, a nitro, a sulphonyl, a C₁-C₄ alkoxy, a C₁-C₄ alkyl, a C₁-C₃ trifluoroalkyl; an amino or a C₁-C₄ alkylamino; acyl; acyloxy; carbamoyl; alkoxycarbonyl; aryloxycarbonyl; cyano; nitro; alkylsulphinyl; arylsulphinyl; sulphamoyl; haloalkyl.

3. Composition according to Claim 2, characterized in that the radicals R₁ in formula (I) are chosen from the group consisting of: hydrogen; C₁-C₄ alkyl; phenyl; phenyl substituted with a chlorine, a sulphonyl, a methoxy, an ethoxy, a C₁-C₄ alkyl or a trifluoromethyl; acyl; cyano; alkoxycarbonyl; carbamoyl; trifluoromethyl.

4. Composition according to Claim 2 or 3, characterized in that the radicals R₁ are chosen from the group consisting of: hydrogen; methyl; ethyl; isopropyl; phenyl; phenyl substituted with a chlorine, a methyl or a methoxy; cyano; methoxycarbonyl; ethoxycarbonyl.

5. Composition according to any one of Claims 1 to 4, characterized in that the radicals R₂ of formula (I) are chosen from the group consisting of: a hydrogen atom; a C₁-C₄ alkoxy; phenoxy; phenoxy substituted with a halogen atom, a C₁-C₄ alkyl, a carboxyl, a trifluoromethyl group; an acyloxy radical; benzyloxy, C₁-C₄ alkylthio; phenylthio; phenylthio substituted with a halogen atom, a C₁-C₄ alkyl, a carboxyl, a trifluoromethyl group; a C₁-C₄ alkylamido; phenylamido; a radical NR^{III}R^{IV} with R^{III} and R^{IV}, which may be identical or different, representing a C₁-C₄ alkyl, a C₁-C₄ hydroxyalkyl; a carboxyl; a C₁-C₄ alkoxycarboxylic radical.

6. Composition according to Claim 5, characterized in that the radicals R₂ of formula (I) are chosen from the group consisting of: hydrogen; chlorine or bromine; methoxy or ethoxy; phenoxy; 4-methylphenoxy; acyloxy; benzyloxy; methylthio or ethylthio; phenylthio; 4-methylphenylthio; 2-tert-butylphenylthio; acetamido; phenylacetamido; dimethylamino; diethylamino; ethylmethylamino; (β-hydroxyethyl)methylamino.

7. Composition according to Claim 6, characterized in that the radicals R₂ of formula (I) are chosen from the group consisting of: hydrogen; chlorine; ethoxy; phenoxy; benzyloxy; acyloxy; acetamido; dimethylamino.

8. Composition according to any one of Claims 1 to 7, characterized in that the radicals R₃ and R₄ of formula (I) are chosen from the group consisting of: acyl; acyloxy; carbamoyl; alkoxycarbonyl; aryloxycarbonyl; cyano; nitro; arylsulphinyl; alkylsulphonyl; arylsulphonyl; haloalkyl; aryl; heteroaryl; C₁-C₄ alkyl; carboxyl; hydrogen.

9. Composition according to Claim 8, characterized in that the radicals R₃ and R₄ are chosen from the group consisting of: alkoxycarbonyl; aryloxycarbonyl; nitro; cyano; arylsulphonyl; carbamoyl; haloalkyl; carboxyl; methyl; ethyl or isopropyl; hydrogen.

10. Composition according to Claim 9, characterized in that the radicals R₃ and R₄ are chosen from the group consisting of: cyano, trifluoromethyl, methoxycarbonyl; ethoxycarbonyl; phenoxycarbonyl, hydrogen; methyl; ethyl or carboxyl.

11. Composition according to any one of Claims 1 to 10, characterized in that the radicals R₅ of formula (I) are chosen from the group consisting of:
a hydrogen atom; a linear or branched C₁-C₄ alkyl; a trifluoromethyl; a phenyl; a phenyl substituted with one or two groups chosen from a halogen, a C₁-C₄ alkyl, a C₁-C₄ alkoxy, a hydroxyl, a carboxyl, a nitro group, a C₁-C₄ alkylthio, a methylenedioxy group, an amino group, a trifluoromethyl group or a C₁-C₄ alkylamino; a benzyl radical; a benzyl radical substituted with a halogen atom, a methyl or isopropyl, methoxy; a C₁-C₄ hydroxyalkyl; a C₁-C₄ aminoalkyl; a C₁-C₄ alkylaminoalkyl; an alkoxy radical chosen from methoxy, ethoxy and phenoxy; methylthio; ethylthio; phenylthio; methanesulphonyl.

12. Composition according to Claim 11, characterized in that the radicals R₅ of formula (I) are chosen from the group consisting of:
hydrogen; an alkyl chosen from methyl, ethyl, isopropyl, n-propyl, tert-butyl; phenyl; toluyl; 2-, 3-or 4-chlorophenyl; 3- or 4-hydroxyphenyl; 3- or 4-aminophenyl; 3- or 4-methoxyphenyl; 4-trifluoromethylphenyl; benzyl; trifluoromethyl; hydroxymethyl; hydroxyethyl; hydroxyisopropyl; aminomethyl or aminoethyl; methoxy or ethoxy; methylthio or ethylthio.

13. Composition according to Claim 12, characterized in that the radicals R₅ of formula (I) are chosen from the group consisting of: hydrogen; methyl; ethyl; isopropyl; phenyl; 4-chlorophenyl; 4-methoxyphenyl; 4-aminophenyl; methoxy or ethoxy; methylthio or ethylthio.

14. Composition according to any one of Claims 1 to 13, characterized in that the compounds of formula (I) are chosen from the group consisting of:
(i) imidazolo[3,2-a]imidazoles of formula: 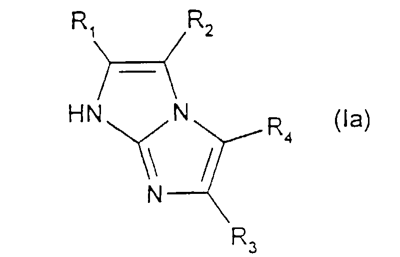
(ii) imidazolo[1,2-b]-1,2,4-triazoles of formula: 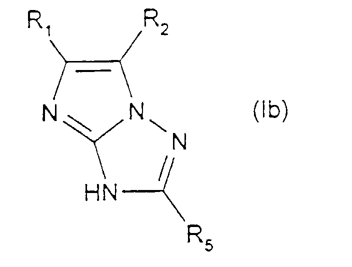 (iii) imidazolo[2,1-c]-1,2,4-triazoles of formula: 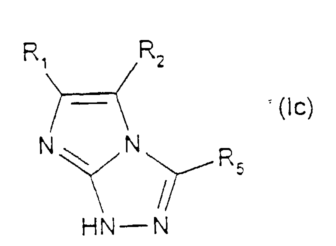 in which R₁, R₂, R₃, R₄ and R₅ have the same meanings as those given above in the preceding claims.

15. Composition according to Claim 14, characterized in that the compounds of formula (Ia) are chosen from those for which:
- R₁ denotes hydrogen, methyl, ethyl, isopropyl or phenyl;
- R₂ denotes hydrogen or chlorine;
- R₃ and R₄ respectively denote cyano and cyano; carboxyl and carboxyl or methoxycarbonyl and cyano.

16. Composition according to Claim 14, characterized in that the compounds of formula (Ib) are chosen from the group consisting of those for which:
- R₁ denotes hydrogen, methyl, ethyl, isopropyl or phenyl;
- R₂ denotes hydrogen or chlorine;
- R₅ denotes hydrogen, methyl, ethyl, phenyl, toluyl or trifluoromethyl.

17. Composition according to Claim 14 characterized in that the compounds of formula (Ic) are chosen from those for which:
- R₁ denotes hydrogen, methyl, ethyl, isopropyl or phenyl;
- R₂ denotes hydrogen or chlorine;
- R₅ denotes hydrogen, methyl, ethyl, phenyl, toluyl or trifluoromethyl.

18. Composition according to Claim 14, characterized in that the compounds of formula (I) are chosen from:
- 7,8-dicyanoimidazolo[3,2-a]imidazole,
- 7,8-dicyano-4-methylimidazolo[3,2-a]imidazole,
- 7,8-dicyano-4-ethylimidazolo[3,2-a]imidazole,
- 7,8-dicyano-4-isopropylimidazolo[3,2-a]imidazole,
- 7,8-dicyano-4-phenylimidazolo[3,2-a]imidazole,
- 5-chloro-7,8-dicyano-4-methylimidazolo[3,2-a]-imidazole,
- 7,8-dicyano-4-trifluoromethylimidazolo[3,2-a]-imidazole,
- imidazolo[1,2-b]-1,2,4-triazole,
- 6-methylimidazolo[1,2-b]-1,2,4-triazole,
- 6-isopropylimidazolo[1,2-b]-1,2,4-triazole,
- 6-phenylimidazolo[1,2-b]-l,2,4-triazole,
- 2,6-dimethylimidazolo[1,2-b]-l,2,4-triazole,
- 6-isopropyl-2-methylimidazolo[1,2-b]-1,2,4-triazole,
- 2-methyl-6-phenylimidazolo[1,2-b]-l,2,4-triazole,
- 6-methyl-2-phenylimidazolo[1,2-b]-1,2,4-triazole,
- 6-isopropyl-2-phenylimidazolo[1,2-b]-1,2,4-triazole,
- 7-chloro-2,6-dimethylimidazolo[1,2-b]-1,2,4-triazole,
- 7-chloro-2-phenyl-6-tertbutylimidazolo[1,2-b]-1,2,4-triazole,
- 6-trifluoromethylimidazolo[1,2-b]-1,2,4-triazole,
- imidazolo[2,1-c]-1,2,4-triazole,
- 5-methylimidazolo[2,1-c]-1,2,4-triazole,
- 5,8-dimethylimidazolo[2,1-c]-l,2,4-triazole,
- 5-methyl-8-phenylimidazolo[2,1-c]-1,2,4-triazole,
- 8-phenylimidazolo[2,1-c]-1,2,4-triazole,
- 6-chloro-5,8-dimethylimidazolo[2,l-c]-l,2,4-triazole,
and the addition salts thereof with an acid.

19. Composition according to any one of the preceding claims, characterized in that the addition salts with an acid for the compounds of formula (I) are chosen from the hydrochlorides, hydrobromides, tartrates, tosylates, benzenesulphonates, sulphates, lactates and acetates.

20. Composition according to any one of the preceding claims, characterized in that the compound(s) of formula (I) represent(s) from 0.0005 to 12 % by weight relative to the total weight of the dye composition.

21. Composition according to claim 20, characterized in that compound(s) of formula (I) represent(s) from 0,005 to 6% by weight relative to the total weight of the dye composition.

22. Composition according to any one of the preceding claims, characterized in that the oxidation base(s) is (are) chosen from para-phenylenediamines, bisphenylalkylenediamines, para-aminophenols, ortho-aminophenols and heterocyclic bases, and the addition salts thereof with an acid.

23. Composition according to any one of the preceding claims, characterized in that the oxidation base(s) represent(s) from 0.0005 to 12% by weight approximately relative to the total weight of the dye composition.

24. Composition according to Claim 23, characterized in that the oxidation base(s) represent(s) from 0.005 to 6% by weight approximately relative to the total weight of the dye composition.

25. Composition according to any one of the preceding claims, characterized in that it also contains one or more additional couplers other than the compounds of formula (I) and/or one or more direct dyes.

26. Composition according to any one of the preceding claims, characterized in that the medium which is suitable for dyeing (or the support) consists of water or a mixture of water and at least one organic solvent chosen from lower C₁-C₄ alkanols, glycerol, glycols and glycol ethers, aromatic alcohols, similar products and mixtures thereof.

27. Composition according to any one of the preceding claims, characterized in that it has a pH of between 3 and 12.

28. Composition according to any one of the preceding claims, characterized in that it is in the form of liquids, creams or gels or is in any other form which is suitable for dyeing keratin fibres, and in particular human hair.

29. Use of the compounds of formula (I) or the addition salts thereof with an acid, as defined in any one of Claims 1 to 19, as couplers in compositions for the oxidation dyeing of keratin fibres, and in particular human keratin fibres such as the hair, in combination with at least one oxidation base.

30. Process for the oxidation dyeing of keratin fibres, and in particular human keratin fibres such as the hair, characterized in that at least one dye composition as defined in any one of Claims 1 to 28 is applied to these fibres, the colour being developed at acidic, neutral or alkaline pH using an oxidizing agent which is added to the dye composition only at the moment of use, or which is present in an oxidizing composition that is applied simultaneously or sequentially in a separate manner.

31. Process according to Claim 30, characterized in that the oxidizing agent is chosen from hydrogen peroxide, urea peroxide, alkali metal bromates and persalts such as perborates and persulphates.

32. Multi-compartment dyeing "kit" or multi-compartment device, in which a first compartment contains a dye composition as defined in any one of Claims 1 to 28, and a second compartment contains an oxidizing composition.
